(19) Europäisches Patentamt
European Patent Office
Office européen des brevets

(11) EP 0 661 260 B1

# (12) FASCICULE DE BREVET EUROPEEN

(45) Date de publication et mention de la délivrance du brevet: **12.03.1997 Bulletin 1997/11**

(21) Numéro de dépôt: **94402551.9**

(22) Date de dépôt: **10.11.1994**

(51) Int Cl.[6]: **C07C 233/87**, C07C 237/20, C07C 237/22, C07C 233/55, C07C 251/48, C07C 233/73, C07C 271/22, A61K 31/16, C07C 317/40, C07D 333/38, C07D 207/34

(54) **Nouveaux composés bi-aromatiques dérivés d'amide, compositions pharmaceutiques et cosmétiques les contenant et utilisations**

Neue von Amiden abgeleitete biaromatische Verbinungen, pharmazeutische und kosmetische Zusammensetzungen, die sie enthalten, und ihre Verwendungen

New bi-aromatic compounds derived from amides pharmaceutical compositions and cosmetic compositions containing them and their use

(84) Etats contractants désignés:
**AT BE CH DE DK ES FR GB GR IE IT LI LU MC NL PT SE**

(30) Priorité: **15.12.1993 FR 9315066**

(43) Date de publication de la demande:
**05.07.1995 Bulletin 1995/27**

(73) Titulaire: **CENTRE INTERNATIONAL DE RECHERCHES DERMATOLOGIQUES GALDERMA, ( CIRD GALDERMA)**
**06560 Valbonne (FR)**

(72) Inventeur: **Bernardon, Jean-Michel**
**F-06650 Le Rouret (FR)**

(74) Mandataire: **Tezier Herman, Béatrice**
**L'OREAL,**
**Département Propriété Industrielle,**
**90, rue du Gal Roguet**
**92583 Clichy Cédex (FR)**

(56) Documents cités:
**EP-A- 0 227 431**
**EP-A- 0 514 264**
**WO-A-92/06948**
**US-A- 3 829 467**

Remarques:
Le dossier contient des informations techniques présentées postérieurement au dépôt de la demande et ne figurant pas dans le présent fascicule.

EP 0 661 260 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Jouve, 75001 PARIS (FR)

## Description

L'invention concerne, à titre de produits industriels nouveaux et utiles, des composés bi-aromatiques dérivés d'amide. Elle concerne également l'utilisation de ces nouveaux composés dans des compositions pharmaceutiques destinées à un usage en médecine humaine ou vétérinaire, ou bien encore dans des compositions cosmétiques.

Il est connu selon la demande WO-A-92 06948 et selon la demande EP-A-0514264 des composés bi-aromatiques qui ont une activité dans les domaines de la différenciation et de la prolifération cellulaire, et trouvent des applications plus particulièrement dans le traitement topique et systémique des affections dermatologiques liées à un désordre de la kératinisation.

Les composés selon l'invention ont une activité marquée dans les domaines de la différenciation et de la prolifération cellulaire, et trouvent des applications plus particulièrement dans le traitement topique et systémique des affections dermatologiques liées à un désordre de la kératinisation, des affections dermatologiques (ou autres) à composante inflammatoire et/ou immunoallergique, et des proliférations dermiques ou épidermiques qu'elles soient bénignes ou malignes. Ces composés peuvent en outre être utilisés dans le traitement des maladies de dégénérescence du tissu conjonctif, pour lutter contre le vieillissement de la peau, qu'il soit photo-induit ou chronologique, et traiter les troubles de la cicatrisation. Ils trouvent par ailleurs une application dans le domaine ophtalmologique, notamment dans le traitement des cornéopathies.

On peut également utiliser les composés selon l'invention dans des compositions cosmétiques pour l'hygiène corporelle et capillaire.

Les composés selon l'invention peuvent être représentés par la formule générale (I) suivante :

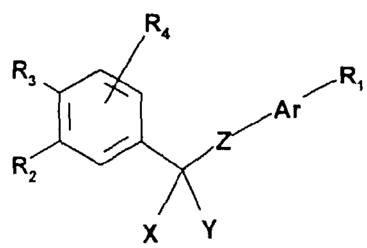

(I)

dans laquelle :

* Z représente -CO-NH- ou -NH-CO-,

* Ar représente un radical choisi parmi les radicaux de formules (a)-(e) suivantes :

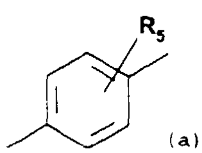
(a)

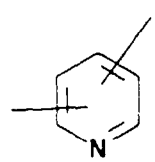
(b)

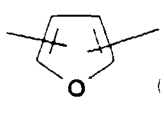
(c)

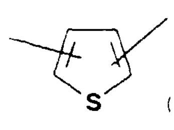
(d)

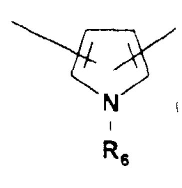
(e)

$R_5$ et $R_6$ ayant la signification donnée ci-après,

* $R_1$ représente :

(i) un atome d'hydrogène

(ii) un radical $-CH_3$

(iii) un radical $-CH_2-O-R_6$

(iv) un radical $-O-R_6$

(v) un radical $-CO-R_7$,

(vi) un radical $-S(O)_tR_9$

$R_6$, $R_7$, $R_9$ et t ayant la signification donnée ci-après,

* X représente un atome d'hydrogène ou un radical alkyle inférieur,

* Y représente :

(i) un radical de formule :

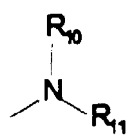

(ii) un radical $-CH_2OR_{12}$

(iii) un radical $-COR_{13}$

(iv) un radical $-(CH_2)_n-COR_{14}$

$R_{10}$, $R_{11}$, $R_{12}$, $R_{13}$, $R_{14}$ et n ayant la signification donnée ci-après,

* $R_2$ et $R_3$ représentent un atome d'hydrogène, un radical alkyle linéaire ou ramifié ayant de 1 à 20 atomes de carbone, un radical $-OR_6$ ou un radical $-SR_6$, $R_6$ ayant la signification donnée ci-après, étant entendu que $R_2$ et $R_3$, pris ensemble, peuvent former avec le cycle aromatique adjacent un cycle à 5 ou 6 chainons éventuellement substitué par des groupes méthyle et/ou éventuellement interrompu par un atome d'oxygène ou de soufre,

* $R_4$ représente un atome d'hydrogène, un atome d'halogène, un radical alkyle inférieur ou un radical $-OR_6$, $R_6$ ayant la signification donnée ci-après,

étant entendu que dans tout ce qui précède :

- $R_5$ a la même signification que $R_4$,

- $R_6$ représente un atome d'hydrogène, un radical alkyle linéaire ou ramifié ayant de 1 à 20 atomes de carbone ou un radical $-CO-R_9$, $R_9$ ayant la signification donnée ci-après,

- $R_7$ représente :

(a) un atome d'hydrogène
(b) un radical alkyle inférieur
(c) un radical de formule :

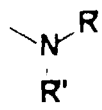

R' et R'' ayant la signification donnée ci-après,
(d) un radical $-OR_8$, $R_8$ ayant la signification donnée ci-après,

- $R_8$ représente un atome d'hydrogène, un radical alkyle linéaire ou ramifié ayant de 1 à 20 atomes de carbone, un radical alkényle, un radical mono ou polyhydroxyalkyle, un radical aryle ou aralkyle éventuellement substitué ou un reste de sucre ou un reste d'aminoacide ou de peptide,
- $R_9$ représente un radical alkyle inférieur,
- $R_{10}$ représente un atome d'hydrogène ou un radical alkyle inférieur,
- $R_{11}$ représente un atome d'hydrogène, un radical alkyle inférieur, un radical $-CO-R_9$ ou un radical $-COOR_9$,
- $R_{12}$ représente un radical $R_6$ ou un radical $-CH_2-O-CH_2-CH_2-O-CH_3$,
- $R_{13}$ représente un atome d'hydrogène ou un radical alkyle inférieur,
- $R_{14}$ représente un radical $-OR_8$ ou un radical de formule

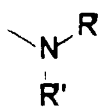

R' et R'' ayant la signification ci-dessous,

- R' et R'' représentent un atome d'hydrogène, un radical alkyle inférieur, un radical mono ou polyhydroxyalkyle, un radical aryle éventuellement substitué ou un reste d'aminoacide ou de peptide ou de sucre ou encore, pris ensemble, forment un hétérocycle,
- t est un nombre entier égal à 0,1 ou 2,
- n est un nombre entier égal à 0 ou 1,
- les radicaux X et Y ci-dessus, pris ensemble, peuvent former un radical unique à double liaison de formule $=N-OR_6$ ou $=CH-COR_{14}$.

L'invention vise également les sels des composés de formule (I) ci-dessus dans le cas où $R_1$ représente une fonction acide carboxylique, ainsi que les isomères optiques et géométriques desdits composés. Lorsque les composés selon l'invention se présentent sous forme de sels, il s'agit de préférence de sels d'un métal alcalin ou alcalino-terreux, ou encore de zinc ou d'une amine organique.

Selon la présente invention, on entend par radical alkyle inférieur un radical ayant de 1 à 6 atomes de carbone, de préférence les radicaux méthyle, éthyle, isopropyle, butyle, tertiobutyle et hexyle.

Par radical alkyle linéaire ou ramifié ayant de 1 à 20 atomes de carbone, on entend notamment les radicaux méthyle, éthyle, propyle, 2-éthyl-hexyle, octyle, dodécyle, hexadécyle et octadécyl.

Par radical monohydroxyalkyle, on entend un radical ayant de préférence 2 ou 3 atomes de carbone, notamment un radical 2-hydroxyéthyle, 2-hydroxypropyle ou 3-hydroxypropyle.

Par radical polyhydroxyalkyle, on entend un radical contenant de préférence de 3 à 6 atomes de carbone et de 2 à 5 groupes hydroxyles tels que les radicaux 2,3-dihydroxypropyle, 2,3,4-trihydroxybutyle, 2,3,4,5-tétrahydroxypentyle ou le reste du pentaérythritol.

Par radical aryle, on entend de préférence un radical phényle éventuellement substitué par au moins un atome

d'halogène, un hydroxyle ou une fonction nitro.

Par radical aralkyle, on entend de préférence le radical benzyle ou phénéthyle éventuellement substitué par au moins un atome d'halogène, un hydroxyle ou une fonction nitro.

Par radical alkényle, on entend un radical contenant de préférence de 1 à 5 atomes de carbone et présentant une ou plusieurs insaturations éthyléniques, tel que plus particulièrement le radical allyle.

Par reste de sucre, on entend un reste dérivant notamment de glucose, de galactose ou de mannose, ou bien encore de l'acide glucuronique.

Par reste d'aminoacide, on entend notamment un reste dérivant de la lysine, de la glycine ou de l'acide aspartique, et par reste de peptide on entend plus particulièrement un reste de dipeptide ou de tripeptide résultant de la combinaison d'acides aminés.

Par hétérocycle enfin, on entend de préférence un radical pipéridino, morpholino, pyrrolidino ou pipérazino, éventuellement substitué en position 4 par un radical alkyle en $C_1$-$C_6$ ou mono ou polyhydroxyalkyle tels que définis ci-dessus.

Lorsque $R_4$ et $R_5$ représente un atome d'halogène, celui-ci est de préférence un atome de fluore, de chlore et de brome.

Lorsque $R_1$ représente -O-$R_6$ , $R_6$ représente, de préférence, un radical alkyle inférieur.

Parmi les composés de formule (I) ci-dessus rentrant dans le cadre de la présente invention, on peut notamment citer les suivants :

- Acide 4-[α-amino-(5,6,7, 8-tétrahydro-5,5,8,8-tétraméthyl-2-naphtyl)acétamido]benzoïque
- Acide 4-[α-méthoximino-(5,6,7,8-tétrahydro-5,5,8,8-tétraméthyl-2-naphtyl)acétamido]benzoïque
- Acide 4-[α-acétamido-(5,6,7,8-tétrahydro-5,5,8,8-tétraméthyl-2-naphtyl)acétamido]benzoïque
- Acide *anti*-4-[α-hydroximino-(5,6,7,8-tétrahydro-5,5,8,8-tétraméthyl-2-naphtyl)acétamido]benzoïque
- Acide *syn*-4-[α-hydroximino-(5,6,7,8-tétrahydro-5,5,8,8-tétraméthyl-2-naphtyl)acétamido]benzoïque
- Acide 4-[α-méthoxycarbonyl-(5,6,7,8-tétrahydro-5,5,8,8-tétraméthyl-2-naphtyl)acétamido]benzoïque
- Acide 4-[α-carboxy-(5,6,7,8-tétrahydro-5,5,8,8-tétraméthyl-2-naphtyl)acétamido]benzoïque
- Acide 4-[α-carbamoyl-(5,6,7,8-tétrahydro-5,5,8,8-tétraméthyl-2-naphtyl)acétamido]benzoïque
- Acide 2-hydroxy-4-[α-*tert*-butoxycarboxamido-(5,6,7,8-tétrahydro-5,5,8,8-tétraméthyl-2-naphtyl)acétamido]benzoïque
- Acide 2-hydroxy-4-[α-amino-(5,6,7,8-tétrahydro-5,5,8,8-tétraméthyl-2-naphtyl)acétamido]benzoïque
- Acide 4-[α-méthoxycarbonyl-(5,6,7,8-tétrahydro-5,5,8,8-tétraméthyl-2-naphtyl)méthylcarbamoyl]benzoïque
- Acide 4-[α-carboxyl-(5,6,7,8-tétrahydro-5,5,8,8-tétraméthyl-2-naphtyl)méthylcarbamoyl]benzoïque
- Acide -4-[α-hydroxyméthyl-(5,6,7,8-tétrahydro-5,5,8,8-tétraméthyl-2-naphtyl)méthylcarbamoyl]benzoïque
- Acide -4-[α-acétoxyméthyl-(5,6,7,8-tétrahydro-5,5,8,8-tétraméthyl-2-naphtyl)méthylcarbamoyl]benzoïque
- Acide syn-4-[α-propyloxyimino-(5,6,7,8-tétrahydro-5,5,8,8-tétraméthyl-2-naphtyl)acétamido]benzoïque
- Acide anti 4-[α-propyloxyimino-(5,6,7,8-tétrahydro-5,5,8,8-tétraméthyl-2-naphtyl)acétamido]benzoïque
- Acide syn-4-[α-hexyloxyimino-(5,6,7,8-tétrahydro-5,5,8,8-tétraméthyl-2-naphtyl)acétamido]benzoïque
- Acide anti 4-[α-hexyloxyimino-(5,6,7,8-tétrahydro-5,5,8,8-tétraméthyl-2-naphtyl)acétamido]benzoïque
- Acide syn-4-[α-heptyloxyimino-(5,6,7,8-tétrahydro-5,5,8,8-tétraméthyl-2-naphtyl)acétamido]benzoïque

- Acide anti 4-[α-heptyloxyimino-(5,6,7,8-tétrahydro-5,5,8,8-tétraméthyl-2-naphtyl)acétamido]benzoïque
- Acide syn-4-[α-nonyloxyimino-(5,6,7,8-tétrahydro-5,5,8,8-tétraméthyl-2-naphtyl)acétamido]benzoïque
- Acide anti 4-[α-nonyloxyimino-(5,6,7,8-tétrahydro-5,5,8,8-tétraméthyl-2-naphtyl)acétamido]benzoïque
- Acide syn-4-[α-dodecyloxyimino-(5,6,7,8-tétrahydro-5,5,8,8-tétraméthyl-2-naphtyl)acétamido]benzoïque
- 5-[a-amino-(5,6,7,8-tétrahydro-5,5,8,8-tétraméthyl-2-naphtyl)acétamido]-2-thiophènecarboxylate de méthyle
- N-méthyl-5-[α-amino-(5,6,7,8-tétrahydro-5,5,8,8-tétraméthyl-2-naphtyl) acétamido]-2-pyrrolecarboxylate de méthyle
- N-méthyl-4-[α-amino-(5,6,7,8-tétrahydro-5,5,8,8-tétraméthyl-2-naphtyl) acétamido]-2-pyrrolecarboxylate de méthyle
- N-propyl-4-[α-amino-(5,6,7,8-tétrahydro-5,5,8,8-tétraméthyl-2-naphtyl) acétamido]-2-pyrrolecarboxylate de méthyle
- 4'-[α-amino-(5,6,7,8-tétrahydro-5,5,8,8-tétraméthyl-2-naphtyl)acétamido] acétophénone
- 4-[α-amino-(5,6,7,8-tétrahydro-5,5,8,8-tétraméthyl-2-naphtyl)toluène
- 4-[α-amino-(5,6,7,8-tétrahydro-5,5,8,8-tétraméthyl-2-naphtyl)phényl sulfonylméthane
- 4-[α-tert-butoxycarboxamido-(5,6,7,8-tétrahydro-5,5,8,8-tétraméthyl-2-naphtyl)acétamido]phénylcarboxamide
- Acétate de 4'-[α-amino-(5,6,7,8-tétrahydro-5,5,8,8-tétraméthyl-2-naphtyl)benzyle
- Acide syn-2-hydroxy-4-[α-hydroxyimino-(5,6,7,8-tétrahydro-5,5,8,8-tétraméthyl-2-naphtyl)acétamido]benzoïque
- Acide 4-[2-éthoxycarbonyl-1-(5,6,7,8-tétrahydro-5,5,8,8-tétraméthyl-2-naphtyl)acrylamido]benzoïque
- 4-[2-tert-butoxycarbonyl-1-(5,6,7,8-tétrahydro-5,5,8,8-tétraméthyl-2-naphtyl) acrylamido]benzoate d'allyle

Selon la présente invention, les composés de formule (I) plus particulièrement préférés sont ceux pour lesquels X représente l'atome d'hydrogène, Y représente le radical $-NH_2$, et $R_7$ représente un radical $-OR_8$, ces deux derniers radicaux ayant la signification donnée ci-avant.

La présente invention a également pour objet les procédés de préparation des composés de formule (I) ci-dessus selon les schémas réactionnels donnés aux Figures 1 et 2.

Les dérivés de formule (Ia) peuvent être préparés par une suite de réactions comprenant la synthèse d'un dérivé de glycine de formule (2) obtenu par la réaction de Strecker-Bücherer à partir d'un aldéhyde aromatique de formule (1) puis protection de la fonction amine sous forme de carbamate (BOC). Le dérivé de formule (2) est couplé avec l'aniline de formule (6) soit par l'intermédiaire du chlorure d'acide ou en présence de dicyclohexylcarbodiimide et de diméthylaminopyridine dans un solvant tel que le THF ou le dichlorométhane.

Les dérivés de formule (Ib) sont préparés à partir de (Ia) par coupure du carbamate soit en présence d'acide trifluoroacétique ou en présence d'iodure de triméthylsilane

Les dérivés de formule (Ic) peuvent être obtenus à partir du composé de formule (2) par réduction de la fonction acide via un anhydride mixte avec un hydrure alcalin (par exemple le borohydrure de sodium). Le composé (3) ainsi obtenu est couplé avec un chlorure de benzoyle de formule (5) dans un solvant organique tel le dichlorométhane ou le THF contenant une amine tertiaire (triéthylamine ou pyridine).

Les composés de formule (Id) peuvent être préparés par une suite de réactions comprenant la lithiation à -78°C d'un acétate de formule (7) avec du diisopropylamidure de lithium puis réaction avec du $CO_2$ dans un solvant organique tel que le THF. Le dérivé de formule (8) ainsi obtenu est couplé avec l'aniline de formule (6) soit par l'intermédiaire du chlorure d'acide ou en présence de dicyclohexylcarbodiimide et de diméthylaminopyridine dans un solvant tel que le THF ou le dichlorométhane.

Les composés de formule (Ie) sont préparés à partir de (Id) par saponification de la fonction ester en présence d'une base, telle l'hydroxyde de sodium ou de lithuim dans un solvant alcoolique ou dans le THF.

Les composés de formule (If) peuvent être obtenus à partir du composé de formule (7) par lithiation avec du diisopropylamidure de lithium puis réaction avec du para-formaldéhyde dans un solvant organique tel que le THF. A partir du dérivé a- hydroxyméthyl ester (9) obtenu en protégeant la fontion alcool sous forme d'éther de méthyle substitué (par exemple 2-méthoxyéthoxyméthyléther (MEM) ou tétrahydropyranyléther) et en saponifiant la fonction ester (hydroxyde de sodium ou de lithuim dans un solvant alcoolique ou dans le THF), on obtient le composé de formule (10). Ce dernier est couplé avec l'aniline de formule (6) soit par l'intermédiaire du chlorure d'acide ou en présence de dicyclohexylcarbodiimide et de diméthylaminopyridine dans un solvant tel que le THF ou le dichlorométhane, puis libération de la fonction alcool en présense d'iodure de triméthylsilane ou d'acide trifluoroacétique.

Lorsque $R_1$ représente le radical -COOH, les composés sont préferentiellement préparés en protégeant $R_1$ par un groupe protecteur de type allylique, benzylique ou tert-butylique. Le passage à la forme libre peut alors être effectué :

- dans le cas d'un groupe protecteur allylique, au moyen d'un catalyseur tel que certains complexes de métaux de transition en présence d'une amine secondaire,
- dans le cas d'un groupe protecteur benzylique, par débenzylation en présence d'hydrogène, au moyen d'un catalyseur tel que le palladium sur du charbon,
- dans le cas d'un groupe protecteur tert-butylique au moyen d'iodure de triméthylsilane.

La présente invention a également pour objet à titre de médicament les composés de formule (I) telle que définie ci-dessus.

Ces composés présentent une activité dans le test de différenciation des cellules (F9) de tératocarcinome embryonnaire de souris (Cancer Research 43, p. 5268, 1983) et/ou dans le test d'inhibition de l'ornithine décarboxylase après induction par le TPA chez la souris (Cancer Research 38, p. 793-801, 1978). Ces tests montrent les activités des composés respectivement dans les domaines de la différenciation et de la prolifération cellulaire.

Les composés selon l'invention conviennent particulièrement bien dans les domaines de traitement suivants :

1) pour traiter les affections dermatologiques liées à un désordre de la kératinisation portant sur la différenciation et sur la prolifération notamment pour traiter les acnées vulgaires, comédoniennes, polymorphes, rosacées, les acnées nodulokystiques, conglobata, les acnées séniles, les acnées secondaires telles que l'acnée solaire, médicamenteuse ou professionnelle,

2) pour traiter d'autres types de troubles de la kératinisation, notamment les ichtyoses, les états ichtyosiformes, la maladie de Darrier, les kératodermies palmoplantaires, les leucoplasies et les états leucoplasiformes, le lichen cutané ou muqueux (buccal),

3) pour traiter d'autres affections dermatologiques liées à un trouble de la kératinisation avec une composante inflammatoire et/ou immuno-allergique et notamment toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale ; les composés peuvent également être utilisés dans certaines affections inflammatoires ne présentant pas de trouble de la kératinisation,

4) pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires,

5) pour traiter d'autres désordres dermatologiques tels que les dermatoses bulleuses et les maladies du collagène,

6) pour traiter certains troubles ophtalmologiques, notamment les cornéopathies,

7) pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique,

8) pour prévenir ou guérir les stigmates de l'atrophie épidermique et/ou dermique induite par les corticostéroïdes locaux ou systémiques, ou toute autre forme d'atrophie cutanée,

9) pour prévenir ou traiter les troubles de la cicatrisation ou pour prévenir ou pour réparer les vergetures,

10) pour lutter contre les troubles de la fonction sébacée tels que l'hyperséborrhée de l'acnée ou la séborrhée simple,

11) dans le traitement ou la prévention des états cancéreux ou précancéreux,

12) dans le traitement d'affections inflammatoires telles que l'arthrite,

13) dans le traitement de toute affection d'origine virale au niveau cutané ou général,

14) dans la prévention ou le traitement de l'alopécie,

15) dans le traitement d'affections dermatologiques ou générales à composante immunologique,

16) dans le traitement d'affections du système cardiovasculaire telles que l'artériosclérose.

Dans les domaines thérapeutiques mentionnés ci-dessus, les composés selon l'invention peuvent être avantageusement employés en combinaison avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques. Par vitamines D ou leurs dérivés, on entend par exemple les dérivés de la vitamine $D_2$ ou $D_3$ et en particulier la 1,25-dihydroxyvitamine $D_3$. Par anti-radicaux libres, on entend par exemple l'α-tocophérol, la Super Oxyde Dismutate, l'Ubiquinol ou certains chélatants de métaux. Par α-hydroxy ou α-céto acides ou leurs dérivés, on entend par exemple les acides lactique, malique, citrique, glycolique, mandélique, tartrique, glycérique ou ascorbique ou leurs sels, amides ou esters. Enfin, par bloqueurs de canaux ioniques, on entend par exemple le Minoxidil (2,4-diamino-6-pipéridino-pyrimidine-3-oxyde) et ses dérivés.

La présente invention a également pour objet des compositions médicamenteuses contenant au moins un composé de formule (I) telle que définie ci-dessus, l'un de ses isomères optiques ou géométriques ou un de ses sels.

La présente invention a donc ainsi pour objet une nouvelle composition médicamenteuse destinée notamment au traitement des affections susmentionnées, et qui est caractérisée par le fait qu'elle comprend, dans un support pharmaceutiquement acceptable et compatible avec le mode d'administration retenu pour cette dernière, au moins un composé de formule (I), l'un de ses isomères optiques ou géométriques ou un de ses sels.

L'administration des composés selon l'invention peut être effectuée par voie entérale, parentérale, topique ou oculaire.

Par voie entérale, les médicaments peuvent se présenter sous forme de comprimés, de gélules, de dragées, de sirops, de suspensions, de solutions, de poudres, de granulés, d'émulsions, de microsphères ou de nanosphères ou de vésicules lipidiques ou polymériques permettant une libération contrôlée. Par voie parentérale, les compositions peuvent se présenter sous forme de solutions ou de suspensions pour perfusion ou pour injection.

Les composés selon l'invention sont généralement administrés à une dose journalière d'environ 0,01 mg/kg à 100 mg/Kg en poids corporel, et ceci à raison de 1 à 3 prises.

Par voie topique, les compositions pharmaceutiques à base de composés selon l'invention sont plus particulièrement destinées au traitement de la peau et des muqueuses et peuvent alors se présenter sous forme d'onguents, de crêmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée.

Ces compositions par voie topique peuvent par ailleurs se présenter soit sous forme anhydre, soit sous une forme aqueuse, selon l'indication clinique.

Par voie oculaire, ce sont principalement des collyres.

Ces compositions à usage topique ou oculaire contiennent au moins un composé de formule (I) telle que définie ci-dessus, ou l'un de ses isomères optiques ou géométriques ou encore l'un de ses sels, à une concentration de préférence comprise entre 0,001% et 5% en poids par rapport au poids total de la composition.

Les composés de formule (I) selon l'invention trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les aspects néfastes du soleil ou dans le traitement des peaux physiologiquement sèches, pour prévenir et/ou pour lutter contre le vieillissement photo-induit ou chronologique.

Dans le domaine cosmétique, les composés selon l'invention peuvent par ailleurs être avantageusement employés en combinaison avec d'autres composés à activité de type rétinoïde, avec les vitamines D ou leurs dérivés, avec des

corticostéroïdes, avec des anti-radicaux libres, des α-hydroxy ou α-céto acides ou leurs dérivés, ou bien encore avec des bloqueurs de canaux ioniques, tous ces différents produits étant tels que définis ci-avant.

La présente invention vise donc également une composition cosmétique qui est caractérisée par le fait qu'elle comprend, dans un support cosmétiquement acceptable et convenant à une application topique, au moins un composé de formule (I) telle que définie ci-dessus ou l'un de ses isomères optiques ou géométriques ou l'un de ses sels, cette composition cosmétique pouvant notamment se présenter sous la forme d'une crème, d'un lait, d'une lotion, d'un gel, de microsphères ou nanosphères ou vésicules lipidiques ou polymériques, d'un savon ou d'un shampooing.

La concentration en composé de formule (I) dans les compositions cosmétiques selon l'invention est avantageusement comprise entre 0,001% et 3% en poids par rapport à l'ensemble de la composition.

Les compositions médicamenteuses et cosmétiques selon l'invention peuvent en outre contenir des additifs inertes ou même pharmacodynamiquement ou cosmétiquement actifs ou des combinaisons de ces additifs, et notamment : des agents mouillants; des agents dépigmentants tels que l'hydroquinone, l'acide azélaïque, l'acide caféïque ou l'acide kojique; des émollients; des agents hydratants comme le glycérol, le PEG 400, la thiamorpholinone, et ses dérivés ou bien encore l'urée; des agents antiséborrhéiques ou antiacnéiques, tels que la S-carboxyméthylcystéine, la S-benzylcystéamine, leurs sels ou leurs dérivés, ou le péroxyde de benzoyle; des antibiotiques comme l'érythromycine et ses esters, la néomycine, la clindamycine et ses esters, les tétracyclines; des agents antifongiques tels que le kétokonazole ou les polyméthylène-4,5 isothiazolidones-3; des agents favorisant la repousse des cheveux, comme le Minoxidil (2,4-diamino-6-pipéridino-pyrimidine-3-oxyde) et ses dérivés, le Diazoxide (7-chloro 3-méthyl 1,2,4-benzothiadiazine 1,1-dioxyde) et le Phénytoïn (5,4-diphénylimidazolidine 2,4-dione); des agents anti-inflammatoires non stéroïdiens; des caroténoïdes et, notamment, le β-carotène; des agents anti-psoriatiques tels que l'anthraline et ses dérivés; et enfin les acides eicosa-5,8,11,14-tétraynoïque et eicosa-5,8,11-trynoïque, leurs esters et amides.

Les compositions selon l'invention peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des antioxydants, tels que l'α-tocophérol, le butylhydroxyanisole ou le butylhydroxytoluène.

On va maintenant donner, à titre d'illustration et sans aucun caractère limitatif, plusieurs exemples d'obtention de composés actifs de formule (I) selon l'invention, ainsi que diverses formulations concrètes à base de tels composés.

**EXEMPLE 1**

*Acide 4-[α-amino-(5,6,7,8-tétrahydro-5,5,8,8-tétraméthyl-2-naphtyl)acétamido]benzoïque*

(a) 4-(5,6,7,8-tétrahydro-5,5,8,8-tétraméthyl-2-naphtyl)-2,5-imidazolinedione

Dans un tricol, on introduit 21,6 g (0,1 mole) de 5,6,7,8-tétrahydro-5,5,8,8-tétraméthyl-2-naphtaldéhyde et 350 ml d'éthanol. On ajoute une solution de 14,7 g (0,3 mole) de cyanure de soduim et 38,4 g (0,4 mole) de carbonate d'ammonium dans 350 ml d'eau et chauffe à 50°C pendant 6 heures. Le milieu réactionnel est concentré à 300 ml par évaporation sous pression réduite, puis extrait avec de l'éther éthylique. On décante la phase organique, lave à l'eau, sèche sur sulfate de magnésium, évapore. On recueille 24,9 g (87%) du produit attendu de point de fusion 213-4°C.

(b) 5,6,7,8-tétrahydro-5,5,8,8-tétraméthyl-2-naphtylglycine

Une solution de 12,9 g (0,045 mole) du produit précédent dans 200 ml de soude à 16% (w/v) est chauffée à reflux pendant 12 heures. Le milieu réactionnel est refroidit, extrait avec de l'acétate d'éthyle, la phase aqueuse est acidifiée à pH 5,5 avec de l'acide chlorhydrique concentré, le solide qui a précipité est filtré, lavé avec de l'acétate d'éthyle et séché sur $P_2O_5$. On recueille 7,5 g (64%) de 5,6,7,8-tétrahydro-5,5,8,8-tétraméthyl-2-naphtylglycine de point de fusion > 360°C.

(c) N-(*tert*-butoxycarbonyl)-5,6,7,8-tétrahydro-5,5,8,8-tétraméthyl-2-naphtylglycine

Dans un ballon, on introduit 6,3 g (24 mmoles) de 5,6,7,8-tétrahydro-5,5,8,8-tétraméthyl-2-naphtylglycine, 25 ml de THF et 50 ml de NaOH (1N). On ajoute 15 g (70 mmoles) de di-*tert*-butyl dicarbonate et agite à température ambiante pendant 12 heures. On extrait le milieu réactionnel avec de l'éther éthylique (3x100 ml), recueille la phase aqueuse, acidifie à pH 2 avec de l'acide chlorhydrique concentré, extrait avec de l'éther éthylique, décante la phase organique, lave à l'eau, sèche sur sulfate de magnésium, évapore. On recueille 7,1 g (82%) du produit attendu.

(d) 4-[α-*tert*-butoxycarboxamido-(5,6,7,8-tétrahydro-5,5,8,8-tétraméthyl-2-naphtyl)acétamido]benzoate de benzyle

Dans un ballon, on introduit 6,1 g (16,9 mmoles) de N-(*tert*-butoxycarbonyl)-5,6,7,8-tétrahydro-5,5,8,8-tétraméthyl-2-naphtylglycine 3,8 g (16,9 mmoles) de 4-aminobenzoate de benzyle et 50 ml de THF. On ajoute successivement 3,5 g (16,9 mmoles) de 1,3-dicyclohexylcarbodiimide, 2,1 g (16,9 mmoles) de 4-diméthylaminopyridine et agite à température ambiante pendant 4 heures. On verse le milieu réactionnel dans l'eau, extrait avec de l'éther éthylique, décante la phase organique, sèche sur sulfate de magnésium, évapore. Le résidu obtenu est purifié par chromatographie sur colonne de silice élué avec un mélange de dichlorométhane et d'hexane (80-20). On recueille 6,1 g (64%) de l'ester attendu.

(e) 4-[α-amino-(5,6,7,8-tétrahydro-5,5,8,8-tétraméthyl-2-naphtyl)acétamido]benzoate de benzyle

Dans un ballon, on introduit 5 g (8,7 mmoles) du produit précédent et 50 ml de dichlorométhane. On refroidit dans la glace et ajoute goutte à goutte 1,25 ml (8,7 mmoles) d'iodure de triméthylsilane et agite à température ambiante pendant 2 heures. On verse le milieu réactionnel dans la glace, extrait avec de l'éther éthylique, décante la phase organique, sèche sur sulfate de magnésium, évapore. Le résidu obtenu est trituré dans 100 ml d'un mélange hexane-éther éthylique (90-10), filtré et séché. On recueille 4,1 g (99%) du produit attendu de point de fusion 196-7°C.

(f) acide 4-[α-amino-(5,6,7,8-tétrahydro-5,5,8,8-tétraméthyl-2-naphtyl)acétamido]benzoïque

Dans un ballon, on introduit 2,9 g (6,1 mmoles) de l'ester précédent, 50 ml de THF, 150 ml d'une solution de soude méthanolique (2N) et chauffe à reflux pendant 2 heures. On évapore à sec le milieu réactionnel, reprend le résidu par l'eau, acidifie à pH 5 avec de l'acide chlorhydrique, filtre le solide qui a précipité, le lave à l'eau, le sèche sur $P_2O_5$. On recueille 1,9 g (83%) d'acide 4-[α-amino-(5,6,7,8-tétrahydro-5,5,8,8-tétraméthyl-2-naphtyl) acétamido]benzoïque de point de fusion 203-4°C.

**EXEMPLE 2**

*Acide 4-[α-méthoximino-(5,6,7,8-tétrahydro-5,5,8,8-tétraméthyl-2-naphtyl)acétamido]benzoïque*

(a) 4-[α-méthoximino-(5,6,7,8-tétrahydro-5,5,8,8-tétraméthyl-2-naphtyl)acétamido]benzoate d'allyle

Dans un ballon, on introduit 1,7 g (4 mmoles) de 4-(5,6,7,8-tétrahydro-5,5,8,8-tétraméthyl-2-naphtylglyoxyloyloxy) benzoate d'allyle (préraration décrite dans la demande de brevet WO 92/06948), 100 ml d'éthanol, 1 g (12 mmoles) de chlorhydrate de méthoxyamine et 120 ml de NaOH (1N). On chauffe à reflux pendant 12 heures, évapore le milieu réactionnel, reprend le résidu par l'eau, extrait avec de l'éther éthylique, décante la phase organique, sèche sur sulfate de magnésium, évapore. Le résidu obtenu est purifié par chromatographie sur colonne de silice élué avec un mélange dichlorométhane-hexane (50-50). Après évaporation des solvants, on recueille 1,5 g (84%) d'ester attendu.

(b) Acide 4-[α-méthoximino-(5,6,7,8-tétrahydro-5,5,8,8-tétraméthyl-2-naphtyl)acétamido]benzoïque

Dans un tricol et sous courant d'azote, on introduit 1,4 g (3,1 mmoles)de l'ester précédent, 190 mg (0,16 mmole) de tétrakis(triphénylphosphine)palladium(0), et 50 ml de THF. On ajoute ensuite goutte à goutte 2,7 ml (31 mmoles) de morpholine et agite à température ambiante pendant 2 heures. On évapore le milieu réactionnel à sec, reprend par l'eau, acidifie à pH 1, extrait avec de l'éther éthylique, décante la phase organique, lave à l'eau, sèche sur sulfate de magnésium, évapore. Le résidu obtenu est trituré dans 50 ml de dichlorométhane, filtré et sèché. On recueille 1,2 g (95%) d'acide 4-[α-méthoximino-(5,6,7,8-tétrahydro-5,5,8,8-tétraméthyl-2-naphtyl)acétamido]benzoïque de point de fusion 208-9°C.

**EXEMPLE 3**

*Acide 4-[α-acétamido-(5,6,7,8-tétrahydro-5,5,8,8-tétraméthyl-2-naphtyl)acétamido]benzoïque*

(a) 4-[α-acétamido-(5,6,7,8-tétrahydro-5,5,8,8-tétraméthyl-2-naphtyl)acétamido]benzoate de benzyle

Dans un ballon, on introduit 1,5 g (3,2 mmoles) de 4-[α-amino-(5,6,7,8-tétrahydro-5,5,8,8-tétraméthyl-2-naphtyl)acétamido]benzoate de benzyle, 75 ml de THF et 490 µl (3,5 mmoles) de triéthylamine. On ajoute goutte à goutte 250 µl (3,5 mmoles) de chlorure d'acétyle et agite à température ambiante 2 heures. On verse le milieu réactionnel dans l'eau, extrait avec de l'acétate d'éthyle, décante la phase organique, sèche sur sulfate de magnésium, évapore. Le résidu obtenu est trituré dans l'hexane, filtré et séché. On obtient 1,6 g (94%) du produit attendu de point de fusion 258-9°C.

(b) Acide 4-[α-acétamido-(5,6,7,8-tétrahydro-5,5,8,8-tétraméthyl-2-naphtyl)acétamido]benzoïque

De manière analogue à l'exemple 1(f) à partir de 1 g (1,9 mmoles) de 4-[α-acétamido-(5,6,7, 8-tétrahydro-5,5,8,8-tétraméthyl-2-naphtyl)acétamido]benzoate de benzyle, on obtient 480 mg (58%) de l'acide attendu de point de fusion 197-8°C

**EXEMPLE 4**

*Acide syn-4[α-hydroximino-(5,6,7,8-tétrahydro-5,5,8,8-tétraméthyl-2-naphtyl)acétamido]benzoïque*

(a) 4-[α-hydroximino-(5,6,7,8-tétrahydro-5,5,8,8-tétraméthyl-2-naphtyl)acétamido]benzoate d'allyle

Dans un ballon, on introduit 1,7 g (4 mmoles) de 4-(5,6,7,8-tétrahydro-5,5,8,8-tétraméthyl-2-naphtylglyoxyloyloxy) benzoate d'allyle 1,1 g (16 mmoles) de chlorhydrate d'hydroxylamine et 50 ml d'éthanol. On ajoute goutte à goutte 16 ml de NaOH (1N) et chauffe à reflux pendant 4 heures. On évapore le milieu réactionnel à sec, reprend le résidu dans l'eau, extrait avec de l'éther éthylique, décante la phase organique, sèche sur sulfate de magnésium, évapore. Le résidu obtenu est chromatographié sur colonne de silice, élué avec un mélange dichlorométhane-éther éthylique (98-2). Après évaporation des solvants, on obtient 1,6 g (94%) du produit attendu de point de fusion 176-7°C.

(b) Acide *syn*-4-[α-hydroximino-(5,6,7,8-tétrahydro-5,5,8,8-tétraméthyl-2-naphtyl)acétamido]benzoïque

De manière analogue à l'exemple 2(b) à partir de 10 g (23 mmoles) de l'ester précédent, on obtient 5,6 g (62%) de l'acide attendu de point de fusion 265-6°C.

**EXEMPLE 5**

*Acide anti 4-[α-hydroximino-(5,6,7,8-tétrahydro-5,5,8,8-tétraméthyl-2 -naphtyl)acétamido]benzoïque*

Dans un réacteur photochimique, on introduit 3 g (7,6 mmoles) d'acide *syn*-4-[α-hydroximino-(5,6,7,8-tétrahydro-5,5,8,8-tétraméthyl-2-naphtyl)acétamido]benzoïque dans 500 ml de méthanol et agite 48 heures à température ambiante en irradiant (lampe Hanovia moyenne pression, sans filtre). On évapore le milieu réactionnel, et purifie le résidu obtenu par chromatographie sur colonne de silice élué avec un mélange dichlorométhaneméthanol (90-10). Après évaporation des solvants, on recueille 1,2 g (40%) d'acide *anti* 4-[α-hydroximino-(5,6,7,8-tétrahydro-5,5,8,8-tétraméthyl-2-naphtyl)acétamido]benzoïque de point de fusion 269-70°C.

**EXEMPLE 6**

*Acide 4-[α-méthoxycarbonyl-(5,6,7,8-tétrahydro-5,5,8,8-tétraméthyl-2-naphtyl)acétamido]benzoïque*

(a) 5,6,7,8-tétrahydro-5,5,8,8-tétraméthyl-2-naphtylacétate de méthyle

Dans un ballon, on introduit 15 g (0.06 mole) d'acide 5,6,7,8-tétrahydro-5,5,8,8-tétraméthyl-2-naphtylacétique, 150 ml de méthanol et 1,6 ml d'acide sulfurique concentré On chauffe à reflux pendant 4 heures, évapore le milieu réactionnel, reprend dans l'eau, extrait avec de l'acétate d'éthyle, décante la phase organique, sèche sur sulfate de magnésium. On recueille 15,9 g (99%) de l'ester attendu de point de fusion 83-5°C.

(b) Acide α-méthoxycarbonyl-(5,6,7,8-tétrahydro-5,5,8,8-tétraméthyl-2-naphtyl)acétique

Dans un tricol et sous courant d'azote, on introduit 2,1 ml ((14,6 mmoles) de diisopropylamine et 80 ml de THF. A -78°C, on ajoute goutte à goutte 5,8 ml (14,6 mmoles) de n-ButylLithium (2,5 M), agite pendant 30' puis ajoute goutte à goutte une solution de 3,2g (12,2 mmoles) de 5,6,7,8-tétrahydro-5,5,8,8-tétraméthyl-2-naphtylacétate de méthyle dissous dans 80 ml de THF et agite pendant une heure. Toujours à -78°C, on introduit du $CO_2$ pendant 30' puis laisse remonter la température à -40°C et verse le milieu réactionnel dans une solution d'acide chlorhydrique 5N. On extrait avec de l'acétate d'éthyle, décante la phase organique, sèche sur sulfate de magnésium, évapore. On recueille 3,7 g (100%) d'acide attendu sous forme d'une huile légèrement jaune.

(c) Chlorure de l'acide α-méthoxycarbonyl-(5,6,7,8-tétrahydro-5,5,8,8-tétraméthyl-2-naphtyl)acétique

Dans un ballon, on introduit 3,1 g (0,01 mole) de l'acide précédent, 30 ml de toluène et 0,2 ml de DMF puis chauffe à 50°C. On ajoute goutte à goutte 900 µl (0,012 mole) de chlorure de thionyle et chauffe à 80°C pendant une heure. On évapore à sec le milieu réactionnel et recueille (100%) du chlorure d'acide brut, qui est utilisé tel quel pour la suite de la synthèse.

(d) 4-[α-méthoxycarbonyl-(5,6,7,8-tétrahydro-5,5,8,8-tétraméthyl-2-naphtyl)acétamido]benzoate d'allyle

Dans un ballon, on introduit 1,8 g (10 mmoles) de 4-aminobenzoate d'allyle, 50 ml de THF et 1,7 ml (12

mmoles) de triéthylamine. On ajoute goutte à goutte une solution de 3,3 g (10 mmoles) de chlorure de l'acide α-méthoxycarbonyl-(5,6,7,8-tétrahydro-5,5,8,8-tétraméthyl-2-naphtyl)acétique dans 25 ml de THF et agite à température ambiante pendant 2 heures. On verse le milieu réactionnel dans l'eau, extrait avec de l'acétate d'éthyle, décante la phase organique, sèche sur sulfate de magnésium, évapore. Le résidu obtenu est recristallisé dans l'éthanol, on recueille après filtration et sèchage 3,3 g (69%) du produit attendu de point de fusion 155-6°C.

(e) Acide 4-[α-méthoxycarbonyl-(5,6,7,8-tétrahydro-5,5,8,8-tétraméthyl-2-naphtyl)acétamido]benzoïque

De manière analogue à l'exemple 2(b) à partir de 3,25 g (7 mmoles) de l'ester allylique précédent, on obtient 2,47 g (83%) de l'acide attendu de point de fusion 156-7°C.

## EXEMPLE 7

*Acide 4-[α-carboxy-(5,6,7,8-tétrahydro-5,5,8,8-tétraméthyl-2-naphtyl)acétamido]benzoïque*

De manière analogue à l'exemple 1(f) à partir de 1,85 g (4 mmoles) de 4-[α-méthoxycarbonyl-(5,6,7,8-tétrahydro-5,5,8,8-tétraméthyl-2-naphtyl)acétamido]benzoate d'allyle, on obtient 1,2 g (74%) d'acide 4-[α-carboxy-(5,6,7,8-tétrahydro-5,5,8,8-tétraméthyl-2-naphtyl)acétamido]benzoïque de point de fusion 213-4°C.

## EXEMPLE 8

*Acide 4-[α-carbamoyl-(5,6,7,8-tétrahydro-5,5,8,8-tétraméthyl-2-naphtyl)acétamido]benzoïque*

(a) 5,6,7,8-tétrahydro-5,5,8,8-tétraméthyl-2-naphtylacétate de benzyle

Dans un tricol et sous courant d'azote, on introduit 730 mg (0,024 mole) d'hydrure de sodium (80%) et 100 ml de DMF, on ajoute goutte à goutte une solution de 5 g (0,02 mole) d'acide 5,6,7,8-tétrahydro-5,5,8,8-tétraméthyl-2-naphtylacétique dans 40 ml de DMF et agite jusqu'a cessation du dégagement gazeux. On ajoute ensuite 2,9 ml (0,024 mole) de bromure de benzyle et agite à température ambiante pendant 4 heures. On verse le milieu réactionnel dans l'eau, extrait avec de l'acétate d'éthyle, décante la phase organique, sèche sur sulfate de magnésium, évapore.On recueille 6,7 g (100%) de l'ester attendu sous forme d'une huile légèrement jaune.

(b) Acide α-benzyloxycarbonyl-(5,6,7,8-tétrahydro-5,5,8,8-tétraméthyl-2 naphtyl)acétique

De manière analogue à l'exemple 6(b) à partir de 14 g (0,041 mole) de l'ester précédent, on obtient 10,8 g (68%) du produit attendu sous forme d'une huile incolore.

(c) Chlorure de l'acide α-benzyloxycarbonyl-(5,6,7,8-tétrahydro-5,5,8,8-tétraméthyl-2-naphtyl)acétique

De manière analogue à l'exemple 6(c) à partir de 5 g (13 mmoles) de l'acide α-benzyloxycarbonyl-(5,6,7,8-tétrahydro-5,5,8,8-tétraméthyl-2-naphtyl)acétique, on obtient 5,2 g (100%) du chlorure d'acide brut qui sera utilisé tel quel pour la suite de la synthèse.

(d) α-carbamoyl-(5,6,7,8-tétrahydro-5,5,8,8-tétraméthyl-2-naphtyl)acétate de benzyle

Dans un ballon, on introduit 125 ml d'ammoniaque (33%) refroidit à 0°C et ajoute goutte à goutte une solution de 5,2 g (13 mmoles) du chlorure d'acide précédent dans 50 ml d'éther éthylique et agite à température ambiante pendant une heure. On verse le milieu réactionnel dans l'eau, extrait avec de l'éther éthylique, décante la phase organique, sèche sur sulfate de magnésium, évapore. Le résidu obtenu est recristallisé dans un mélange acétate d'éthylehexane (30-70), on obtient 3,9 g (79%) du produit attendu de point de fusion 112-3°C.

(e) Acide α-carbamoyl-(5,6,7,8-tétrahydro-5,5,8,8-tétraméthyl-2-naphtyl)acétique

Dans un réacteur, on introduit 3,9 g (10 mmoles) de α-carbamoyl-(5,6,7,8-tétrahydro-5,5,8,8-tétraméthyl-2-naphtyl)acétate de benzyle, 1,2 g de palladium sur charbon (10%) et 100 ml de dioxanne. On hydrogène à température ambiante et sous une pression de 7 bars pendant 4 heures, filtre sur celite et évapore à sec le filtrat. Le résidu obtenu est repris dans le minimum d'éther éthylique, filtré et sèché. On recueille 2,2 g (76%) de l'acide attendu.

(f) 4-[α-carbamoyl-(5,6,7,8-tétrahydro-5,5,8,8-tétraméthyl-2-naphtyl)acétamido] benzoate d'allyle

De manière analogue à l'exemple1(d) par réaction de 2 g (6,9 mmoles) de l'acide précédent avec 1,22 g (6,9 mmoles) de 4-aminobenzoate d'allyle, on obtient 1,78 g (57%) de l'ester attendu de point de fusion 210-1°C.

(g) Acide 4-[α-carbamoyl-(5,6,7,8-tétrahydro-5,5,8,8-tétraméthyl-2-naphtyl)acétamido]benzoïque

De manière analogue à l'exemple 2(b) à partir de 1,6 g (3,6 mmoles) de 4-[α-carbamoyl-(5,6,7,8-tétrahydro-5,5,8,8-tétraméthyl-2-naphtyl)acétamido]benzoate d'allyle, on obtient 690 mg (47%) de l'acide attendu de point de fusion 165-7°C.

**EXEMPLE 9**

*Acide 2-hydroxy-4-[α-tert-butoxycarboxamido-(5,6,7,8-tétrahydro-5,5,8,8-tétraméthyl-2-naphtyl)acétamido]benzoïque*

(a) 2-hydroxy-4-[α-*tert*-butoxycarboxamido-(5,6,7,8-tétrahydro-5,5,8,8-tétraméthyl-2-naphtyl)acétamido]benzoate d'allyle

De manière analogue à l'exemple 1(d) par réaction de 8 g (22 mmoles) de N-(*tert*-butoxycarbonyl)-5,6,7,8-tétrahydro-5,5,8,8-tétraméthyl-2-naphtylglycine préparé à l'exemple 1(c) avec 4,3 g (22 mmoles) de 2-hydroxy-4-aminobenzoate d'allyle, on obtient 2,5 g (20%) de l'ester allylique attendu sous forme d'une huile.

(b) Acide 2-hydroxy-4-[α-*tert*-butoxycarboxamido-(5,6,7,8-tétrahydro-5,5,8,8-tétraméthyl-2-naphtyl)acétamido] benzoïque

De manière analogue à l'exemple 2(b) à partir de 2,4 g (4,5 mmoles) de 2-hydroxy-4-[α-*tert*-butoxycarboxamido-(5,6,7,8-tétrahydro-5,5,8,8-tétraméthyl-2-naphtyl)acétamido]benzoate d'allyle, on obtient 1,9 g (85%) de l'acide attendu de point de fusion 160-5°C.

**EXEMPLE 10**

*Acide 2-hydroxy-4-[α-amino-(5,6,7,8-tétrahydro-5,5,8,8-tétraméthyl-2-naphtyl)acétamido]benzoïque*

Dans un ballon, on introduit 1,52 g (3 mmoles) de l'acide précédent et 15 ml de dichlorométhane, on ajoute goutte à goutte 880 µl (6 mmoles) d'iodure de triméthylsilane et agite à température ambiante pendant 4 heures. On verse le milieu réactionnel dans l'eau, extrait avec de l'acétate d'éthyle, décante la phase organique, lave à l'eau, sèche sur sulfate de magnésium, évapore. Le résidu obtenu est trituré dans le minimum d'éther éthylique, filtré, sèché. On recueille 930 mg (77%) du produit attendu de point de fusion 240-1°C.

**EXEMPLE 11**

*Acide 4-[α-méthoxycarbonyl-(5,6,7,8-tétrahydro-5,5,8,8-tétraméthyl-2-naphtyl)méthylcarbamoyl]benzoïque*

(a) 5,6,7,8-tétrahydro-5,5,8,8-tétraméthyl-2-naphtylglycinate de méthyle

Dans un ballon, on introduit 8 g (22 mmoles) de 5,6,7,8-tétrahydro-5,5,8,8-tétraméthyl-2-naphtylglycine, 100 ml de méthanol et ajoute goutte à goutte 9 ml (0,12 mole) de chlorure de thionyle. On agite à température ambiante pendant 12 heures, évapore le milieu réactionnel, reprend le résidu par l'eau bicarbonatée et l'éther éthylique, décante la phase organique, sèche sur sulfate de magnésium, évapore. On recueille 6 g (100%) de l'ester attendu.

(b) 4-[α-méthoxycarbonyl-(5,6,7,8-tétrahydro-5,5,8,8-tétraméthyl-2-naphtyl)méthylcarbamoyl]benzoate d'allyle

De manière analogue à l'exemple 6(d) par réaction de 1,1 g (4,8 mmoles) de chlorure de 4-allyloxycarbonylbenzoyle (préparé selon la demande de brevet WO 92/06948) avec 1,2 g (4,4 mmoles) de 5,6,7,8-tétrahydro-5,5,8,8-tétraméthyl-2-naphtylglycinate de méthyle, on obtient 1,6 g (78%) de l'ester allylique attendu sous forme d'une huile légèrement jaune.

(c) Acide 4-[α-méthoxycarbonyl-(5,6,7,7,8-tétrahydro-5,5,8,8-tétraméthyl-2-naphtyl)méthylcarbamoyl]benzoïque

De manière analogue à l'exemple 2(b) à partir de 1,6 g (3,4 mmoles) de 4-[α-méthoxycarbonyl-(5,6,7,8-tétrahydro-5,5,8,8-tétraméthyl-2-naphtyl)méthylcarbamoyl]benzoate d'allyle, on obtient 750 mg (52%) de l'acide attendu de point de fusion 95-100°C.

**EXEMPLE 12**

*Acide 4-[α-carboxyl-(5,6,7,8-tétrahydro-5,5,8,8-tétraméthyl-2-naphtyl)méthylcarbamoyl]benzoïque*

De manière analogue à l'exemple 1(f) à partir de 1,1 g (2,2 mmoles) de 4-[α-méthoxycarbonyl-(5,6,7,8-tétrahydro-5,5,8,8-tétraméthyl-2-naphtyl)méthylcarbamoyl]benzoate d'allyle, on obtient 860 mg (92%) de l'acide attendu de point

de fusion 180-5°C.

**EXEMPLE 13**

*Acide -4-[α-hydroxyméthyl-(5,6,7,8-tétrahydro-5,5,8,8-tétraméthyl-2-naphtyl)méthylcarbamoyl]benzoïque*

a) 2-*tert*-butoxycarboxamido-2-(5,6,7,8-tétrahydro-5,5,8,8-tétraméthyl-2-naphtyl)éthanol

Dans un tricol et sous courant d'azote, on introduit 38 g (0,105 mole) de N-(*tert*-butoxycarbonyl)-5,6,7,8-tétrahydro-5,5,8,8-tétraméthyl-2-naphtylglycine [préparé à l'exemple 1(c)], 14,7 ml (0,105 mole) de triéthylamine et 160 ml de THF. On refroidit à 0°C et ajoute goutte à goutte 9,9 ml (0,105 mole) de chloroformiate d'éthyle et agite à température ambiante pendant 30'. On introduit ensuite une solution de 9,9 g (0,262 mole) de borohydrure de sodium dans 200 ml d'eau et agite à température ambiante pendant 2 heures. On verse le milieu réactionnel dans la glace, extrait avec de l'acétate d'éthyle, décante la phase organique, sèche sur sulfate de magnésium, évapore. On triture le solide obtenu dans l'hexane, filtre, sèche et recueille 26 g (72%) de 2-*tert*-butoxycarboxamido-2-(5,6,7,8-tétrahydro-5,5,8,8-tétraméthyl-2-naphtyl)éthanol de point de fusion 119-21°C.

(b) 4-{[2-*tert*-butoxycarboxamido-2-(5,6,7,8-tétrahydro-5,5,8,8-tétraméthyl-2-naphtyl)éthoxy]carbonyl}benzoate d'allyle

De manière anlogue à l'exemple 6(d) par réaction de 1,1g (3,1 mmoles) de l'alcool précédent avec 690 mg (3,4 mmoles) de chlorure de 4-allyoxycarbonylbenzoyle, on obtient 1,36 g (85%) de 4-{[2-*tert*-butoxycarboxamido-2-(5,6,7,8-tétrahydro-5,5,8,8-tétraméthyl-2-naphtyl)éthoxy]carbonyl}benzoate d'allyle

(c) 4-[α-hydroxyméthyl-(5,6,7,8-tétrahydro-5,5,8,8-tétraméthyl-2-naphtyl)méthylcarbamoyl]benzoate d'allyle

Dans un ballon, on introduit 4 g (7,5 mmoles) de l'ester précédent, 40 ml de dichlorométhane et ajoute goutte à goutte 10 ml d'acide trifluoroacétique. On agite à température ambiante pendant 8 heures, évapore le milieu réactionnel et recueille le 4-{[2-amino-2-(5,6,7,8-tétrahydro-5,5,8,8-tétraméthyl-2-naphtyl)éthoxy]carbonyl}benzoate d'allyle. Ce produit par chauffage à 70°C dans 50 ml de toluène se réarrange et on obtient 1,07 g (33%) de 4-[α-hydroxyméthyl-(5,6,7,8-tétrahydro-5,5,8,8-tétraméthyl-2-naphtyl)méthylcarbamoyl]benzoate d'allyle de point de fusion 140-5°C.

(d) Acide -4-[α-hydroxyméthyl-(5,6,7,8-tétrahydro-5,5,8,8-tétraméthyl-2-naphtyl)méthylcarbamoyl]benzoïque

De manière analogue à l'exemple 2(b) à partir de 1,05 g (2,5 mmoles) de l'ester allylique précédent, on obtient 660 mg (69%) de l'acide attendu de point de fusion 145-50°C.

**EXEMPLE 14**

*Acide 4-[α-acétoxyméthyl-(5,6,7,8-tétrahydro-5,5,8,8-tétraméthyl-2-naphtyl)méthylcarbamoyl]benzoïque*

(a) 4-[α-acétoxyméthyl-(5,6,7,8-tétrahydro-5,5,8,8-tétraméthyl-2-naphtyl)méthylcarbamoyl]benzoate d'allyle

Dans un ballon, on introduit 395 mg (0,9 mmoles) de 4-[α-hydroxyméthyl-(5,6,7,8-tétrahydro-5,5,8,8-tétraméthyl-2-naphtyl)méthylcarbamoyl]benzoate d'allyle, 15 ml de pyridine et 200 µl (1,8 mmoles) d'anhydride acétique. On agite à température ambiante pendant 8 heures, verse le milieu réactionnel dans l'eau, extrait avec de l'éther éthylique, décante la phase organique, lave à l'eau, sèche sur sulfate de magnésium, évapore.On recueille 435 mg (100%) du produit attendu sous forme d'une huile incolore.

(b) Acide 4-[α-acétoxyméthyl-(5,6,7,8-tétrahydro-5,5,8,8-tétraméthyl-2-naphtyl)méthylcarbamoyl]benzoïque

De manière analogue à l'exemple 2(b) à partir de 606 mg (1,27 mmoles) de 4-[α-acétoxyméthyl-(5,6,7,8-tétrahydro-5,5,8,8-tétraméthyl-2-naphtyl)méthylcarbamoyl]benzoate d'allyle, on obtient 347 mg (60%) de l'acide attendu de point de fusion 95-100°C.

**EXEMPLE 15**

*Acide syn-4-[α-propyloxyimino-(5,6,7,8-tétrahydro-5,5,8,8-tétraméthyl-2-naphtyl)acétamido]benzoïque.*

(a) *syn*-4-[α-propyloxyimino-(5,6,7,8-tétrahydro-5,5,8,8-tétraméthyl-2-naphtyl) acétamido]benzoate d'allyle.

Dans un tricol et sous courant d'azote, on introduit 2,2 g (5 mmoles) de 4-[α-hydroxyimino-(5,6,7,8-tétrahydro-5,5,8,8-tétraméthyl-2-naphtyl)acétamido]benzoate d'allyle [préparé à l'exemple 4(a)] 700 mg (5 mmoles) de carbonate de potassium et 100 ml de méthyléthylcétone. On ajoute 490 µL (5 mmoles) de 1-iodopropane et chauffe

à reflux pendant deux heures. On évapore à sec, reprend par l'eau et l'éther éthylique, décante la phase organique, sèche sur sulfate de magnésium, évapore. Le résidu obtenu est purifié par chromatographie sur colonne de silice élué avec de l'hexane. On recueille 1,4 g (59%) de *syn*-4-[α-propyloxyimino-(5,6,7,8-tétrahydro-5,5,8,8-tétraméthyl-2-naphtyl)acétamido]benzoate d'allyle sous forme d'une huile incolore et 230 mg (10%) de *anti*-4-[α-propyloxyimino-(5,6,7,8-tétrahydro-5,5,8,8-tétraméthyl-2-naphtyl)acétamido]benzoate d'allyle de point de fusion 120-1°C.

(b) acide *syn*-4-[α-propyloxyimino-(5,6,7,8-tétrahydro-5,5,8,8-tétraméthyl-2-naphtyl)acétamido]benzoïque.

Dans un tricol et sous courant d'azote, on introduit 1,2 g (2,5 mmoles) de *syn*-4-[α-propyloxyimino-(5,6,7,8-tétrahydro-5,5,8,8-tétraméthyl-2-naphtyl)acétamido] benzoate d'allyle 100 ml de THF et 100 mg (0,08 mmole) de tétrakis (triphénylphosphine)palladium(0). On ajoute ensuite goutte à goutte 1,1 ml (12,5 mmoles) de morpholine et agite à température ambiante pendant 2 heures. On évapore le milieu réactionnel à sec, reprend par l'eau, acidifie à pH 1, extrait avec de l'éther éthylique, décante la phase organique, lave à l'eau, sèche sur sulfate de magnésium, évapore. Le résidu obtenu est purifié par chromatographie sur colonne de silice élué avec du dichlorométhane. Après évaporation des solvants. On recueille 820 mg (75%) d'acide *syn*-4-[α-propyloxyimino-(5,6,7,8-tétrahydro-5,5,8,8-tétraméthyl-2-naphtyl)acétamido]benzoïque de point de fusion 204-5°C.

**EXEMPLE 16**

*Acide anti 4-[α-propyloxyimino-(5,6,7,8-tétrahydro-5,5,8,8-tétraméthyl-2-naphtyl)acétamido]benzoïque.*

De manière analogue à l'exemple 15(b) à partir de 200 mg (0,4 mmole) d'*anti*-4-[α-propyloxyimino-(5,6,7,8-tétrahydro-5,5,8,8-tétraméthyl-2-naphtyl)acétamido] benzoate d'allyle préparé à l'exemple 15(a), on obtient 130 mg (71%) d'acide *anti* 4-[α-propyloxyimino-(5,6,7,8-tétrahydro-5,5,8,8-tétraméthyl-2-naphtyl)acétamido] benzoïque de point de fusion 215-7°C.

**EXEMPLE 17**

*Acide syn-4-[α-hexyloxyimino-(5,6,7,8-tétrahydro-5,5,8,8-tétraméthyl-2-naphtyl)acétamido]benzoïque.*

(a) *syn*-4-[α-hexyloxyimino-(5,6,7,8-tétrahydro-5,5,8,8-tétraméthyl-2-naphtyl) acétamido]benzoate d'allyle.

De manière analogue à l'exemple 15(a) par réaction de 2,2 g (5 mmoles) de 4-[α-hydroxyimino-(5,6,7,8-tétrahydro-5,5,8,8-tétraméthyl-2-naphtyl)acétamido] benzoate d'allyle avec 750 µl (5 mmoles) de 1-iodohexane, on obtient après chromatographie sur colonne de silice élué avec un mélange de dichlorométhane et d'hexane (20-80) 1,6 g (62%) de *syn*-4-[α-hexyloxyimino-(5,6,7,8-tétrahydro-5,5,8,8-tétraméthyl-2-naphtyl)acétamido]benzoate d'allyle sous forme d'une huile jaune et 420 mg (16%) d'*anti*-4-[α-hexyloxyimino-(5,6,7,8-tétrahydro-5,5,8,8-tétraméthyl-2-naphtyl)acétamido]benzoate d'allyle sous forme d'une huile jaune.

(b) acide *syn*-4-[α-hexyloxyimino-(5,6,7,8-tétrahydro-5,5,8,8-tétraméthyl-2-naphtyl)acétamido]benzoïque.

De manière analogue à l'exemple 15(b) à partir de 1,5 g (2,9 mmoles) de *syn*-4-[α-hexyloxyimino-(5,6,7,8-tétrahydro-5,5,8,8-tétraméthyl-2-naphtyl)acétamido] benzoate d'allyle, on obtient 1,1 g (79%) d'acide *syn*-4-[α-hexyloxyimino-(5,6,7,8-tétrahydro-5,5,8,8-tétraméthyl-2-naphtyl)acétamido]benzoïque de point de fusion 188-90°C.

**EXEMPLE 18**

*Acide anti 4-[α-hexyloxyimino-(5,6,7,8-tétrahydro-5,5,8,8-tétraméthyl-2-naphtyl)acétamido]benzoïque.*

De manière analogue à l'exemple 15(b) à partir de 400 mg (0.77 mmole) d'*anti*-4-[α-hexyloxyimino-(5,6,7,8-tétrahydro-5,5,8,8-tétraméthyl-2-naphtyl)acétamido]benzoate d'allyle préparé à l'exemple 17(a), on obtient 250 mg (68%) d'acide *anti* 4-[α-hexyloxyimino-(5,6,7,8-tétrahydro-5,5,8,8-tétraméthyl-2-naphtyl)acétamido]benzoïque de point de fusion 154-6°C.

**EXEMPLE 19**

*Acide syn-4-[α-heptyloxyimino-(5,6,7,8-tétrahydro-5,5,8,8-tétraméthyl-2-naphtyl)acétamido]benzoïque.*

(a) *syn*-4-[α-heptyloxyimino-(5,6,7,8-tétrahydro-5,5,8,8-tétraméthyl-2-naphtyl)acétamido]benzoate d'allyle.

De manière analogue à l'exemple 15(a) par réaction de 3,25 g (7,5 mmoles) de 4-[α-hydroxyimino-(5,6,7,8-tétrahydro-5,5,8,8-tétraméthyl-2-naphtyl) acétamido]benzoate d'allyle avec 1,2 ml (7,5 mmoles) de 1-iodoheptane, on obtient après chromatographie sur colonne de silice élué avec un mélange de dichlorométhane et d'hexane (50-50) 2 g (50%) de *syn*-4-[α-heptyloxyimino-(5,6,7,8-tétrahydro-5,5,8,8-tétraméthyl-2-naphtyl)acétamido]benzoate d'allyle sous forme d'une huile incolore et 600 mg (15%) d'*anti*-4-[α-heptyloxyimino-(5,6,7,8-tétrahydro-5,5,8,8-tétraméthyl-2-naphtyl)acétamido]benzoate d'allyle sous forme d'une huile jaune.

(b) acide *syn*-4-[α-heptyloxyimino-(5,6,7,8-tétrahydro-5,5,8,8-tétraméthyl-2-naphtyl)acétamido]benzoïque.

De manière analogue à l'exemple 15(b) à partir de 2 g (3,7 mmoles) de *syn*-4-[α-heptyloxyimino-(5,6,7,8-tétrahydro-5,5,8,8-tétraméthyl-2-naphtyl)acétamido] benzoate d'allyle, on obtient 1,5 g (81%) d'acide *syn*-4-[α-heptyloxyimino-(5,6,7,8-tétrahydro-5,5,8,8-tétraméthyl-2-naphtyl)acétamido]benzoïque de point de fusion 178-80°C.

**EXEMPLE 20**

*Acide anti 4-[α-heptyloxyimino-(5,6,7,8-tétrahydro-5,5,8,8-tétraméthyl-2-naphtyl)acétamido]benzoïque.*

De manière analogue à l'exemple 15(b) à partir de 600 mg (1,1 mmole) d'*anti*-4-[α-heptyloxyimino-(5,6,7,8-tétrahydro-5,5,8,8-tétraméthyl-2-naphtyl)acétamido] benzoate d'allyle préparé à l'exemple 19(a), on obtient 250 mg (45%) d' acide *anti* 4-[α-heptyloxyimino-(5,6,7,8-tétrahydro-5,5,8,8-tétraméthyl-2-naphtyl)acétamido] benzoïque de point de fusion 174-8°C.

**EXEMPLE 21**

*Acide syn -4-[α-nonyloxyimino-(5,6,7,8-tétrahydro-5,5,8,8-tétraméthyl-2-naphtyl)acétamido]benzoïque.*

(a) *syn*-4-[α-nonyloxyimino-(5,6,7,8-tétrahydro-5,5,8,8-tétraméthyl-2-naphtyl) acétamido]benzoate d'allyle.

De manière analogue à l'exemple 15(a) par réaction de 3.25 g (7,5 mmoles) de 4-[α-hydroxyimino-(5,6,7,8-tétrahydro-5,5,8,8-tétraméthyl-2-naphtyl)acétamido] benzoate d'allyle avec 1,5 ml (7,5 mmoles) de 1-iodoheptane, on obtient après chromatographie sur colonne de silice élué avec un mélange de dichlorométhane et d'hexane (40-60) 2,8 g (67%) de *syn*-4-[α-nonyloxyimino-(5,6,7,8-tétrahydro-5,5,8,8-tétraméthyl-2-naphtyl)acétamido]benzoate d'allyle sous forme d'une huile jaune et 600 mg (14%) d' *anti*-4-[α-nonyloxyimino-(5,6,7,8-tétrahydro-5,5,8,8-tétraméthyl-2-naphtyl)acétamido]benzoate d'allyle sous forme d'une huile jaune.

(b) acide *syn*-4-[α-nonyloxyimino-(5,6,7,8-tétrahydro-5,5,8,8-tétraméthyl-2-naphtyl)acétamido]benzoïque.

De manière analogue à l'exemple 15(b) à partir de 2,8 g (5 mmoles) de *syn*-4-[α-nonyloxyimino-(5,6,7,8-tétrahydro-5,5,8,8-tétraméthyl-2-naphtyl)acétamido] benzoate d'allyle , on obtient 1,9 g (73%) d'acide *syn*-4-[α-nonyloxyimino-(5,6,7,8-tétrahydro-5,5,8,8-tétraméthyl-2-naphtyl)acétamido]benzoïque de point de fusion 162-3°C.

**EXEMPLE 22**

*Acide anti 4-[α-nonyloxyimino-(5,6,7,8-tétrahydro-5,5,8,8-tétraméthyl-2-naphtyl)acétamido]benzoïque.*

De manière analogue à l'exemple 15(b) à partir de 600 mg (1 mmole) d' *anti*-4-[α-nonyloxyimino-(5,6,7,8-tétrahydro-5,5,8,8-tétraméthyl-2-naphtyl)acétamido] benzoate d'allyle préparé à l'exemple 21(a), on obtient 65 mg (12%) d'acide *anti* 4-[α-nonyloxyimino-(5,6,7,8-tétrahydro-5,5,8,8-tétraméthyl-2-naphtyl)acétamido] benzoïque de point de fusion 155-8°C.

**EXEMPLE 23**

*Acide syn-4-[α-dodecyloxyimino-(5,6,7,8-tétrahydro-5,5,8,8-tétraméthyl-2-naphtyl)acétamido]benzoïque.*

(a) *syn*-4-[α-dodecyloxyimino-(5,6,7,8-tétrahydro-5,5,8,8-tétraméthyl-2-naphtyl) acétamido]benzoate d'allyle.

De manière analogue à l'exemple 15(a) par réaction de 3 g (6,9 mmoles) de 4-[α-hydroxyimino-(5,6,7,8-tétrahydro-5,5,8,8-tétraméthyl-2-naphtyl)acétamido benzoate d'allyle avec 1,7 ml (6,9 mmoles) de 1-bromododécane, on obtient après chromatographie sur colonne de silice élué avec un mélange de dichlorométhane et d'heptane (30-70) 2,3 g (55%) de *syn*-4-[α-dodecyloxyimino-(5,6,7,8-tétrahydro-5,5,8,8-tétraméthyl-2-naphtyl)acétamido] benzoate d'allyle sous forme d'une huile jaune.

(b) acide *syn*-4-[α-dodecyloxyimino-(5,6,7,8-tétrahydro-5,5,8,8-tétraméthyl-2-naphtyl)acétamido]benzoïque

De manière analogue à l'exemple 15(b) à partir de 2,3 g (3,8 mmoles) de *syn*-4-[α-dodecyloxyimino-(5,6,7,8-tétrahydro-5,5,8,8-tétraméthyl-2-naphtyl)acétamido] benzoate d'allyle, on obtient 1,4 g (65%) d'acide *syn*-4-[α-dodecyloxyimino-(5,6,7,8-tétrahydro-5,5,8,8-tétraméthyl-2-naphtyl)acétamido]benzoïque de point de fusion 165-6°C.

**EXEMPLE 24**

*5-[α-amino-(5,6,7,8-tétrahydro-5,5,8,8-tétraméthyl-2-naphtyl)acétamido]-2-thiophènecarboxylate de méthyle.*

(a) 5-amino-2-thiophènecarboxylate de méthyle.

Dans un tricol, on introduit successivement 3,6 g de poudre de fer 1,4 g de $FeSO_4,7H_20$ 25 ml de méthanol et 8 ml d'eau. On ajoute ensuite 940 mg (5 mmoles) de 5-nitro-2-thiophènecarboxylate de méthyle et chauffe à 70°C durant huit heures.On filtre le milieu réactionnel sur célite, évapore le filtrat, reprend par l'eau et l'éther éthylique, décante la phase organique, sèche sur sulfate de magnésium, évapore. Le résidu obtenu est purifié par chromatographie sur colonne de silice élué avec du dichlorométhane, on obtient 2 g (77%) de produit attendu de point de fusion 110-2°C.

(b) 5-[α-*tert*-butoxycarboxamido-(5,6,7,8-tétrahydro-5,5,8,8-tétraméthyl-2-naphtyl) acétamido]-2-thiophènecarboxylate de méthyle.

Dans un ballon, on introduit 1,8 g (5 mmoles) de N-(*tert*-butoxycarbonyl)-5,6,7,8-tétrahydro-5,5,8,8-tétraméthyl-2-naphtylglycine 780 mg (5 mmoles) de 5-amino-2-thiophènecarboxylate de méthyle et 50 ml de THF. On ajoute successivement 1 g (5 mmoles) de 1,3-dicyclohexylcarbodiimide, 610 mg (5 mmoles) de 4-diméthylaminopyrydine et agite à température ambiante pendant 4 heures. On verse le milieu réactionnel dans l'eau, extrait avec de l'éther éthylique, décante la phase organique, sèche sur sulfate de magnésium, évapore. Le résidu obtenu est purifié par chromatographie sur colonne de silice élué avec l'éther éthylique. On recueille 1,1 g (44%) de l'ester attendu de point de fusion 125-6°C.

(c) 5-[α-amino-(5,6,7,8-tétrahydro-5,5,8,8-tétraméthyl-2-naphtyl)acétamido]-2-thiophènecarboxylate de méthyle.

Dans un ballon, on introduit 1,1 g (2,2 mmoles) de 5-[α-*tert*-butoxy carboxamido-(5,6,7,8-tétrahydro-5,5,8,8-tétraméthyl-2-naphtyl)acétamido]-2-thiophènecarboxylate de méthyle et 50 ml de dichlorométhane. On refroidit dans la glace et ajoute goutte à goutte 3,4 ml (44 mmoles) d'acide trifluoroacétique et agite à température ambiante pendant 2 heures. On verse le milieu réactionnel dans la glace, extrait avec de l'éther éthylique, décante la phase organique, sèche sur sulfate de magnésium, évapore. Le résidu obtenu est chromatographié sur colonne de silice élué avec de l'éther éthylique, on obtient 650 mg (74%) de 5-[α-amino-(5,6,7,8-tétrahydro-5,5,8,8-tétraméthyl-2-naphtyl)acétamido]-2-thiophènecarboxylate de méthyle de point de fusion 177-9°C.

**EXEMPLE 25**

*N-méthyl-5-[α-amino-(5,6,7,8-tétrahydro-5,5,8,8-tétraméthyl-2-naphtyl)acétamido]-2-pyrrolecarboxylate de méthyle.*

(a) 2-trichloroacétylpyrrole.

Dans un tricol, on introduit 45 g (247 mmoles) de chlorure de trichloroacétyle et 100 ml d'éther éthylique. On ajoute goutte à goutte une solution de 15,4 g (230 mmoles) de pyrrole dans 100ml d'éther éthylique et agite à température ambiante une heure, on ajoute ensuite lentement une solution de 20 g de carbonate de potassium dans 60 ml d'eau. On décante la phase organique, sèche sur sulfate de magnésium, évapore, triture le résidu dans l'hexane et filtre. On recueille 42,7 g (87%) de produit attendu de point de fusion 78-9°C.

(b) 5-nitro-2-trichloroacétylpyrrole.

Dans un ballon, on introduit 21,3 g (0,1 mole) de 2-trichloroacétylpyrrole et 100 ml d'anhydride acétique. A 0°C, on ajoute goutte à goutte une solution de 4,2 ml d'acide nitrique fumant 8 ml d'acide acétique 8 ml d'anhydride acétique et laisse remonter à température ambiante. On verse le milieu réactionnel dans la glace, ajuste à pH 8 avec du bicarbonate de sodium, extrait avec du dichlorométhane, décante la phase organique, sèche sur sulfate de magnésium, évapore. Le résidu obtenu est purifié par chromatographie sur colonne de silice élué avec un mélange de dichlorométhane et d'hexane (60-40). On recueille 5,5 g (21%) de 5-nitro-2-trichloroacétylpyrrole sous forme d'une huile vert foncé et 11,2 g de 4-nitro-2-trichloroacétylpyrrole de point de fusion 160-3°C.

(c) 5-nitro-2-pyrrolecarboxylate de méthyle.

Dans un ballon, on introduit 5,5 g (21,3 mmoles) de 5-nitro-2-trichloro acétylpyrrole et 100 ml de méthanol et

ajoute par petites quantités 3.6 g de méthylate de sodium. On agite à température ambiante pendant deux heures, évapore le milieu réactionnel, triture le résidu dans l'eau, filtre le solide et sèche. On recueille 3 g (83%) de l'ester méthylique attendu de point de fusion 178-9°C.

(d) N-méthyl-5-nitro-2-pyrrolecarboxylate de méthyle.

Dans un tricol et sous courant d'azote, on introduit 220 mg (7,6 mmoles) d'hydrure de sodium (80% dans l'huile) et 50 ml de DMF. On ajoute goutte à goutte une solution de 1,3 g (7,6 mmoles) de 5-nitro-2-pyrrolecarboxylate de méthyle dans 50 ml de DMF et agite jusqu'à cessation du dégagement gazeux. A 0°C on ajoute ensuite 480 µl (7,6 mmoles) d'iodométhane et agite pendant quatre heures. On verse le milieu réactionnel dans l'eau, extrait avec de l'éther éthylique, décante la phase organique, sèche sur sulfate de magnésium, évapore. Le résidu obtenu est trituré dans l'hexane, filtré, sèché. On recueille 1,3 g (92%) du produit attendu de point de fusion 117-8°C.

(e) N-méthyl-5-amino-2-pyrrolecarboxylate de méthyle.

Dans un réacteur on introduit 1,31 g (7,4 mmoles) de N-méthyl-5-nitro-2-pyrrolecarboxylate de méthyle, 400 mg de palladium sur charbon (10%) et 50 ml de méthanol. On hydrogène à température ambiante et sous une pression de 7 bars pendant 2 heures. On filtre le catalyseur, lave deux fois avec 25 ml de méthanol, évapore les filtrats. On recueille 1,1 g (100% de l'amine attendu sous forme d'une huile rouge foncé.

(f) N-méthyl-5-[α-*tert*-butoxycarboxamido-(5,6,7,8-tétrahydro-5,5,8,8-tétraméthyl-2-naphtyl)acétamido]-2-pyrrolecarboxylate de méthyle.

De manière analogue à l'exemple 24(b) par réaction de 2,6 g (7,13 mmoles) de N-(*tert*-butoxycarbonyl)-5,6,7,8-tétrahydro-5,5,8,8-tétraméthyl-2-naphtylglycine avec 1,1 g (7,13 mmoles) de N-méthyl-5-amino-2-pyrrolecarboxylate de méthyle, on obtient 600 mg (17%) de l'ester méthylique attendu de point de fusion 185-7°C.

(g) N-méthyl-5-[α-amino-(5,6,7,8-tétrahydro-5,5,8,8-tétraméthyl-2-naphtyl) acétamido]-2-pyrrolecarboxylate de méthyle.

Dans un ballon, on introduit 480 mg (1 mmole) de N-méthyl-5-[α-*tert*-butoxycarboxamido-(5,6,7,8-tétrahydro-5,5,8,8-tétraméthyl-2-naphtyl)acétamido]-2-pyrrolecarboxylate de méthyle et 20 ml de dichlorométhane. On refroidit dans la glace et ajoute goutte à goutte 170µl (1,2 mmoles) d'iodure de triméthylsilane et agite à température ambiante pendant 2 heures. On verse le milieu réactionnel dans la glace, extrait avec de l'éther éthylique, décante la phase organique, sèche sur sulfate de magnésium, évapore. Le résidu obtenu est purifié par chromatographie sur colonne de silice élué avec un mélange d'acétate d'éthyle et d'heptane (70-30). On recueille 210 mg (53%) de N-méthyl-5-[α-amino-(5,6,7,8-tétrahydro-5,5,8,8-tétraméthyl-2-naphtyl)acétamido]-2-pyrrolecarboxylate de méthyle de point de fusion 143-4°C.

**EXEMPLE 26**

*N-méthyl-4-[α-amino-(5,6,7,8-tétrahydro-5,5,8,8-tétraméthyl-2-naphtyl)acétamido]-2-pyrrolecarboxylate de méthyle.*

(a) 4-nitro-2-pyrrolecarboxylate de méthyle.

De manière analogue à l'exemple 25(c) à partir de 11,2 g (43,5 mmoles) de 4-nitro-2-trichloroacétylpyrrole préparé à l'exemple 25(b), on obtient 4,9 g (66%) de l'ester méthylique attendu de point de fusion 196-7°C.

(b) N-méthyl-4-nitro-2-pyrrolecarboxylate de méthyle.

De manière analogue à l'exemple 25(d) par réaction de 2,9 g (17 mmoles) de 4-nitro-2-pyrrolecarboxylate de méthyle avec 1,1 ml d'iodométhane, on obtient 2,8 g (89%) de produit attendu de point de fusion 122-4°C.

(c) N-méthyl-4-amino-2-pyrrolecarboxylate de méthyle.

De manière analogue à l'exemple 25(e) à partir de 2,8 g (15,2 mmoles) de N-méthyl-4-nitro-2-pyrrolecarboxylate de méthyle, on obtient 2,3 g (100%) de l'amine attendu sous forme d'une huile marron.

(d) N-méthyl-4-[α-*tert*-butoxycarboxamido-(5,6,7,8-tétrahydro-5,5,8,8-tétraméthyl-2-naphtyl)acétamido]-2-pyrrolecarboxylate de méthyle.

De manière analogue à l'exemple 24(b) par réaction de 5,4 g (14,9 mmoles) de N-(*tert*-butoxycarbonyl)-5,6,7,8-tétrahydro-5,5,8,8-tétraméthyl-2-naphtylglycine avec 2,3 g (14,9 mmoles) de N-méthyl-4-amino-2-pyrrolecarboxylate de méthyle, on obtient 3,58 g (50%) de l'ester méthylique attendu.

(e) N-méthyl-4-[α-amino-(5,6,7,8-tétrahydro-5,5,8,8-tétraméthyl-2-naphtyl) acétamido]-2-pyrrolecarboxylate de méthyle.

De manière analogue à l'exemple 25(g) à partir de 3,5 g (7,2 mmoles) de N-méthyl-4-[α-*tert*-butoxycarboxamido-(5,6,7,8-tétrahydro-5,5,8,8-tétraméthyl-2-naphtyl)acétamido]-2-pyrrolecarboxylate de méthyle, on obtient 1,35 g (47%) du produit attendu de point de fusion 220-4°C.

**EXEMPLE 27**

*N-propyl-4-[α-amino-(5,6,7,8-tétrahydro-5,5,8,8-tétraméthyl-2-naphtyl)acétamido]-2-pyrrolecarboxylate de méthyle.*

(a) N-propyll-4-nitro-2-pyrrolecarboxylate de méthyle.

De manière analogue à l'exemple 25(d) par réaction de 1,79 g (10,5 mmoles) de 4-nitro-2-pyrrolecarboxylate de méthyle avec 1,23 ml (12,6 mmoles) de 3-iodo propane, on obtient 1,42 g (64%) du produit attendu.

(b) N-propyl-4-amino-2-pyrrolecarboxylate de méthyle.

De manière analogue à l'exemple 25(e) à partir de 1,41 g (6,6 mmoles) de N-propyll-4-nitro-2-pyrrolecarboxylate de méthyle, on obtient 1,18 g (100%) de l'amine attendu sous forme d'une huile marron.

(c) N-propyl-4-[α-*tert*-butoxycarboxamido-(5,6,7,8-tétrahydro-5,5,8,8-tétraméthyl-2-naphtyl)acétamido]-2-pyrrolecarboxylate de méthyle.

De manière analogue à l'exemple 24(b) par réaction de 2,34 g (6,5 mmoles) de N-(*tert*-butoxycarbonyl)-5,6,7,8-tétrahydro-5,5,8,8-tétraméthyl-2-naphtylglycine avec 1,18 g (6,5 mmoles) de N-propyl-4-amino-2-pyrrolecarboxylate de méthyle, on obtient 2,1 g de l'ester méthylique attendu.

(d) N-propyl-4-[α-amino-(5,6,7,8-tétrahydro-5,5,8,8-tétraméthyl-2-naphtyl)acétamido]-2-pyrrolecarboxylate de méthyle.

De manière analogue à l'exemple 25(g) à partir de 2 g (3,8 mmoles) de N-propyl-4-[α-*tert*-butoxycarboxamido-(5,6,7,8-tétrahydro-5,5,8,8-tétraméthyl-2-naphtyl)acétamido]-2-pyrrolecarboxylate de méthyle, on obtient 820 mg (51%) du produit attendu de point de fusion 152-3°C.

**EXEMPLE 28**

*4'-[α-amino-(5,6,7,8-tétrahydro-5,5,8,8-tétraméthyl-2-naphtyl)acétamido]acétophénone.*

(a) 4'-[α-*tert*-butoxycarboxamido-(5,6,7,8-tétrahydro-5,5,8,8-tétraméthyl-2-naphtyl)acétamido]acétophénone.

De manière analogue à l'exemple 24(b) par réaction de 1,8 g (5 mmoles) de N-(*tert*-butoxycarbonyl)-5,6,7,8-tétrahydro-5,5,8,8-tétraméthyl-2-naphtylglycine avec 670 mg (5 mmoles) de 4'-aminoacétophénone, on obtient 2,38 g (42%) de produit attendu de point de fusion 138-9°C.

(b) 4'-[α-amino-(5,6,7,8-tétrahydro-5,5,8,8-tétraméthyl-2-naphtyl)acétamido] acétophénone.

De manière analogue à l'exemple 24(c) à partir de 840 mg (1,7 mmole) de 4'-[α-*tert*-butoxycarboxamido-(5,6,7,8-tétrahydro-5,5,8,8-tétraméthyl-2-naphtyl) acétamido]acétophénone, on obtient 580 mg (87%) du produit attendu de point de fusion 214-5°C.

**EXEMPLE 29**

*4-[α-amino-(5,6,7,8-tétrahydro-5,5,8,8-tétraméthyl-2-naphtyl)toluène.*

(a) 4-[α-*tert*-butoxycarboxamido-(5,6,7,8-tétrahydro-5,5,8,8-tétraméthyl-2-naphtyl)acétamido]toluène.

De manière analogue à l'exemple 24(b) par réaction de 1,8 g (5 mmoles) de N-(*tert*-butoxycarbonyl)-5,6,7,8-tétrahydro-5,5,8,8-tétraméthyl-2-naphtylglycine avec 530 mg (5 mmoles) de 4-aminotoluène, on obtient 1 g (44%) de produit attendu de point de fusion 195-6°C.

(b) 4-[α-amino-(5,6,7,8-tétrahydro-5,5,8,8-tétraméthyl-2-naphtyl)acétamido]toluène

De manière analogue à l'exemple 24(c) à partir de 700 mg (1,55 mmoles) de 4-[α-*tert*-butoxycarboxamido-(5,6,7,8-tétrahydro-5,5,8,8-tétraméthyl-2-naphtyl) acétamido]toluène, on obtient 300 mg (55%) de produit attendu de point de fusion 161-3°C.

**EXEMPLE 30**

*4-[α-amino-(5,6,7,8-tétrahydro-5,5,8,8-tétraméthyl-2-naphtyl)phényl sulfonylméthane.*

(a) 4-[α-*tert*-butoxycarboxamido-(5,6,7,8-tétrahydro-5,5,8,8-tétraméthyl-2-naphtyl)acétamido]phénylsulfonylméthane.

De manière analogue à l'exemple 24(b) par réaction de 1,8 g (5 mmoles) de N-(*tert*-butoxycarbonyl)-5,6,7,8-tétrahydro-5,5,8,8-tétraméthyl-2-naphtylglycine avec 850 mg (5 mmoles) de 4-aminophénylsulfonylméthane, on obtient 300 mg (12%) de produit attendu de point de fusion 156-7°C.

(b) 4'-[α-amino-(5,6,7,8-tétrahydro-5,5,8,8-tétraméthyl-2-naphtyl)acétamido]phénylsulfonylméthane.

De manière analogue à l'exemple 24(c) à partir de 300 mg (0,6 mmole) de 4-[α-*tert*-butoxycarboxamido-(5,6,7,8-tétrahydro-5,5,8,8-tétraméthyl-2-naphtyl) acétamido]phénylsulfonylméthane, on obtient 45 mg (19%) du produit attendu de point de fusion 175-6°C.

**EXEMPLE 31**

*4-[α-tert-butoxycarboxamido-(5,6,7,8-tétrahydro-5,5,8,8-tétraméthyl-2-naphtyl)acétamido]phénylcarboxamide.*

De manière analogue à l'exemple 24(b) par réaction de 1,8 g (5 mmoles) de N-(*tert*-butoxycarbonyl)-5,6,7,8-tétrahydro-5,5,8,8-tétraméthyl-2-naphtylglycine avec 680 mg (5 mmoles) de 4-aminophénylcarboxamide, on obtient 1,5 g (63%) de 4-[α-tert-butoxycarboxamido-(5,6,7,8-tétrahydro-5,5,8,8-tétraméthyl-2-naphtyl)acétamido] phénylcarboxamide de point de fusion > 360°C.

**EXEMPLE 32**

*Acétate de 4'-[α-amino-(5,6,7,8-tétrahydro-5,5,8,8-tétraméthyl-2-naphtyl)benzyle.*

(a) Acétate de 4-nitrobenzyle.

Dans un ballon, on introduit 7,6 g (0,05 mole) d'alcool 4-nitrobenzylique 4,7 ml (0,05 mole) d'anhydride acétique 5,8 ml (0,1 mole) d'acide acétique et chauffe à reflux pendant huit heures. On verse le milieu réactionnel dans l'eau, extrait avec de l'éther éthylique, décante la phase organique, sèche sur sulfate de magnésium, évapore. Le résidu est trituré dans l'hexane, filtré, sèché. On recueille 6,1 g (62%) d'acétate de 4-nitrobenzyle de point de fusion 77-8°C.

(b) acétate de 4-aminobenzyle.

De manière analogue à l'exemple 25(e) à partir de 3 g (15,4 mmoles) d'acétate de 4-nitrobenzyle, on obtient 2,5 g (97%) d'acétate de 4-aminobenzyle de point de fusion 83-4°C.

(c) acétate de 4'-[α-*tert*-butoxycarboxamido-(5,6,7,8-tétrahydro-5,5,8,8-tétraméthyl-2-naphtyl)acétamido]benzyle.

De manière analogue à l'exemple 24(b) par réaction de1,8 g (5 mmoles) de N-(*tert*-butoxycarbonyl)-5,6,7,8-tétrahydro-5,5,8,8-tétraméthyl-2-naphtylglycine avec 820 mg (5 mmoles) d'acétate de 4-aminobenzyle, on obtient 400 mg (16%) du produit attendu de point de fusion 209-10°C.

(d) acétate de 4'-[α-amino-(5,6,7,8-tétrahydro-5,5,8,8-tétraméthyl-2-naphtyl) acétamido]benzyle.

De manière analogue à l'exemple 24(c) à partir de 2,3 g (4,5 mmoles) d'acétate de 4'-[α-*tert*-butoxycarboxamido-(5,6,7,8-tétrahydro-5,5,8,8-tétraméthyl-2-naphtyl)acétamido]benzyle, on obtient 1,3 g (71%) du produit attendu de point de fusion 185-6°C.

**EXEMPLE 33**

*Acide syn-2-hydroxy-4-[α-hydroxyimino-(5,6,7,8-tétrahydro-5,5,8,8-tétraméthyl-2-naphtyl)acétamido]benzoïque.*

(a) 2-hydroxy-4-(5,6,7,8-tétrahydro-5,5,8,8-tétraméthyl-2-naphtoylcarboxamido) benzoate d'allyle.

A une solution de 7,33 g (38 mmoles) de 2-hydroxy-4-aminobenzoate d'allyle 9,2 ml de pyridine et 50 ml de THF, on ajoute goutte à goutte une solution de 10,6 g (38 mmoles) de chlorure de 5,6,7,8-tétrahydro-5,5,8,8-tétraméthyl-2-naphtyl glyoxyloyle (préparation décrite dans le brevet WWO 92/06948) dans 50 ml de THF et agite à température ambiante pendant 4 heures. On verse le milieu réactionnel dans l'eau,extrait avec de l'éther éthylique, décante la phase organique, sèche sur sulfate de magnésium, évapore. Le résidu obtenu est purifié par chromatographie sur colonne de silice élué avec un mélange de dichlorométhane et d'heptane (40-60). On recueille 4,99 g (34%) de 2-hydroxy-4-(5,6,7,8-tétrahydro-5,5,8,8-tétraméthyl -2-naphtoylcarboxamido) benzoate d'allyle de point de fusion 100-1°C.

(b) 2-hydroxy-4-[α-hydroxyimino-(5,6,7,8-tétrahydro-5,5,8,8-tétraméthyl-2-naphtyl)acétamido]benzoate d'allyle.

De manière analogue à l'exemple 4(a) par réaction de 4,6 g (10,5 mmoles) de 2-hydroxy-4-(5,6,7,8-tétrahydro-5,5,8,8-tétraméthyl-2-naphtoylcarboxamido) benzoate d'allyle avec 2,3 g (42,2 mmoles) de chlorhydrate d'hydroxylamine, on obtient 2,5 g (53%) du produit attendu de point de fusion 108-9°C.

(c) Acide *syn*-2-hydroxy-4-[a-hydroxyimino-(5,6,7,8-tétrahydro-5,5,8,8-tétraméthyl-2-naphtyl)acétamido]benzoïque.

De manière analogue à l'exemple 2(b) à partir de 2 g (4,4 mmoles) de 2-hydroxy-4-[a-hydroxy imino-(5,6,7,8-tétrahydro-5,5,8,8-tétraméthyl-2-naphtyl) acétamido]benzoate d'allyle, on obtient après chromatographie sur colonne de silice élué avec un mélange de dichlorométhane ety de méthanol (95-5) 310 mg 15%) d'acide *syn*-2-hydroxy-4-[a-hydroxyimino-(5,6,7,8-tétrahydro-5,5,8,8-tétraméthyl-2-naphtyl)acétamido]benzoïque de point de fusion 240°C avec décomposition.

**EXEMPLE 34**

*Acide 4-[2-éthoxycarbonyl-1-(5,6,7,8-tétrahydro-5,5,8,8-tétraméthyl-2-naphtyl) acrylamido]benzoïque.*

(a) acide 2-éthoxycarbonyl-1-(5,6,7,8-tétrahydro-5,5,8,8-tétraméthyl-2-naphtyl) acrylique.

Dans un tricol et sous courant d'azote on introduit 320 mg (11 mmoles) d'hydrure de sodium (80% dans l'huile) et 20 ml de THF, on ajoute goutte à goutte une solution de 2,2 ml (11 mmoles) de triéthylphosphonoacétate et agite jusqu'à cessation du dégagement gazeux. Cette solution est ajoutée goutte à goutte à une solution de 2,8 g (10 mmoles) du sel de sodium de l'acide 5,6,7,8-tétrahydro-5,5,8,8-tétraméthyl-2-naphtylglyoxylique dans 50 ml de THF. On agite à température ambiante 2 heures, verse le milieu réactionnel dans 100 ml d'acide chlorhydrique 0,5N, extrait avec de l'éther éthylique, décante la phase organique, sèche sur sulfate de magnésium, évapore. Le résidu obtenu est purifié par chromatographie sur colonne de silice élué avec du dichlorométhane. On recueille 2 g (61%) d'acide 2-éthoxycarbonyl-1-(5,6,7,8-tétrahydro-5,5,8,8-tétraméthyl-2-naphtyl)acrylique de point de fusion 114-6°C.

(b) 4-[2-éthoxycarbonyl-1-(5,6,7,8-tétrahydro-5,5,8,8-tétraméthyl-2-naphtyl) acrylamido]benzoate d'allyle.

De manière analogue à l'exemple 24(b) par réaction de 2 g (6 mmoles) d'acide 2-éthoxycarbonyl-1-(5,6,7,8-tétrahydro-5,5,8,8-tétraméthyl-2-naphtyl) acrylique avec 1 g (6 mmoles) de 4-aminobenzoate d'allyle, on obtient 650 mg (22%) de l'ester allylique attendu de point de fusion 116-8°C.

(c) acide 4-[2-éthoxycarbonyl-1-(5,6,7,8-tétrahydro-5,5,8,8-tétraméthyl-2-naphtyl) acrylamido]benzoïque.

De manière analogue à l'exemple 15(b) à partir de 610 mg (1,2 mmole) de 4-[2-éthoxycarbonyl-1-(5,6,7,8-tétrahydro-5,5,8,8-tétraméthyl-2-naphtyl) acrylamido] benzoate d'allyle, on obtient 360 mg (64%) d'acide attendu de point de fusion 155-7°C.

**EXEMPLE 35**

*4-[2-tert-butoxycarbonyl-1-(5,6,7,8-tétrahydro-5,5,8,8-tétraméthyl-2-naphtyl)acrylamido]benzoate d'allyle.*

(a) acide 2-tert-butoxycarbonyl-1-(5,6,7,8-tétrahydro-5,5,8,8-tétraméthyl-2-naphtyl)acrylique.

De manière analogue à l'exemple 34(a) par réaction de 14,8 g (52,3 mmoles) du sel de sodium de l'acide 5,6,7,8-tétrahydro-5,5,8,8-tétraméthyl-2-naphtyl glyoxylique avec 11,4 ml (52,3 mmoles) de P,P-diméthylphosphonoacétate de tert-butyle, on obtient 8,8 g (48%) d'acide 2-tert-butoxycarbonyl-1-(5,6,7,8-tétrahydro-5,5,8,8-tétraméthyl-2-naphtyl)acrylique de point de fusion 134-6°C.

(b) 4-[2-tert-butoxycarbonyl-1-(5,6,7,8-tétrahydro-5,5,8,8-tétraméthyl-2-naphtyl) acrylamido]benzoate d'allyle.

De manière analogue à l'exemple 24(b) par réaction de 4 g (11,1 mmoles) d'acide 2-ter-butoxycarbonyl-1-(5,6,7,8-tétrahydro-5,5,8,8-tétraméthyl-2-naphtyl) acrylique avec 2 g (11,2 mmoles) de 4-aminobenzoate d'allyle, on obtient 1,1 g (20%) de l'ester allylique attendu de point de fusion 155-7°C.

**EXEMPLE 36**

Dans cet exemple, on a illustré diverses formulations concrètes à base des composés selon l'invention.

A- VOIE ORALE

(a) Comprimé de 0,2 g

- Composé préparé à l'exemple 0,001 g
- Amidon 0,114 g
- Phosphate bicalcique 0,020 g
- Silice 0,020 g
- Lactose 0,030 g
- Talc 0,010 g
- Stéarate de magnésium 0,005 g

(b) Suspension buvable en ampoules de 5 ml

- Composé préparé à l'exemple 0,001 g
- Glycérine 0,500 g
- Sorbitol à 70% 0,500 g
- Saccharinate de sodium 0,010 g
- Parahydroxybenzoate de méthyle 0,040 g
- Arome qs
- Eau purifiée qsp 5 ml

(c) Comprimé de 0,8 g

- Composé de l'exemple 10 0,500 g
- Amidon prégélatinisé 0,100 g
- Cellulose microcristalline 0,115 g
- Lactose 0,075 g
- Stéarate de magnésium 0,010 g

(d) Suspension buvable en ampoules de 10 ml

- Composé de l'exemple 4 0,200 g
- Glycérine 1,000g
- Sorbitol à 70% 1,000g
- Saccharinate de sodium 0,010 g
- Parahydroxybenzoate de méthyle 0,080 g
- Arome qs
- Eau purifiée qsp 10 ml

B- VOIE TOPIQUE

(a) Onguent

- Composé de l'exemple 1 0,020 g
- Myristate d'isopropyle 81,700 g
- Huile de vaseline fluide 9,100 g
- Silice ("Aérosil 200" vendue par DEGUSSA) 9,180 g

(b) Onguent

- Composé de l'exemple 10 0,300 g
- Vaseline blanche codex 100 g

(c) Crème Eau-dans-Huile non ionique

- Composé de l'exemple 5 0,100 g
- Mélange d'alcools de lanoline émulsifs, de cires et d'huiles ("Eucerine anhydre" vendu par BDF) 39,900 g
- Parahydroxybenzoate de méthyle 0,075 g
- Parahydroxybenzoate de propyle 0,075 g
- Eau déminéralisée stérile qsp 100 g

(d) Lotion

- Composé de l'exemple 6 0,100 g
- Polyéthylène glycol (PEG 400) 69,900 g
- Ethanol à 95% 30,000 g

(e) Onguent hydrophobe

- Composé de l'exemple 7 0,300 g
- Mirystate d'isopropyle 36,400 g
- Huile de silicone ("Rhodorsil 47 V 300" vendu par RHONE-POULENC) 36,400 g
- Cire d'abeille 13,600 g
- Huile de silicone ("Abil 300.000 cst" vendu par GOLDSCHMIDT) 100g

(f) Crème Huile-dans-Eau non ionique

- Composé de l'exemple 10 1,000 g
- Alcool cétylique 4,000 g
- Monostéarate de glycérole 2,500 g
- Stéarate de PEG 50 2,500 g
- Beurre de karité 9,200 g
- Propylène glycol 2,000 g
- Parahydroxybenzoate de méthyle 0,075 g
- Parahydroxybenzoate de propyle 0,075 g
- Eau déminéralisée stérile 100 g

**Revendications**

**1.** Composés bi-aromatiques dérivés d'amide, caractérisés par le fait qu'ils répondent à la formule générale (I) suivante :

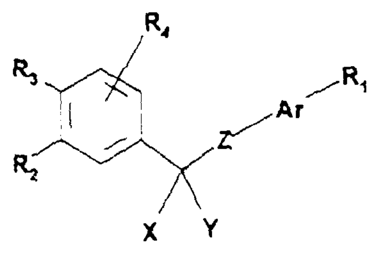

(I)

dans laquelle :

* Z représente -CO-NH- ou -NH-CO-,

* Ar représente un radical choisi parmi les radicaux de formules (a)-(e) suivantes :

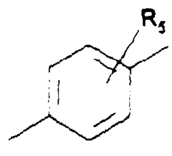 (a) 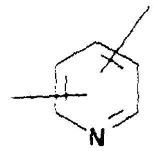 (b)

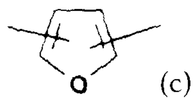 (c) 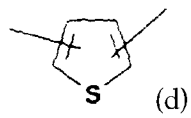 (d) 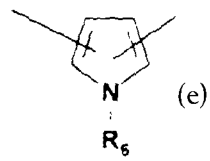 (e)

$R_5$ et $R_6$ ayant la signification donnée ci-après,

* $R_1$ représente :

  (i) un atome d'hydrogène

  (ii) un radical $-CH_3$

  (iii) un radical $-CH_2-O-R_6$

  (iv) un radical $-O-R_6$

  (v) un radical $-CO-R_7$,

  (vi) un radical $-S(O)_tR_9$

  $R_6$, $R_7$, $R_9$ et t ayant la signification donnée ci-après,

* X représente un atome d'hydrogène ou un radical alkyle inférieur,

* Y représente :

  (i) un radical de formule :

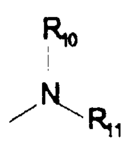

  (ii) un radical $-CH_2OR_{12}$

  (iii) un radical $-COR_{13}$

  (iv) un radical $-(CH_2)_n-COR_{14}$

  $R_{10}$, $R_{11}$, $R_{12}$, $R_{13}$, $R_{14}$ et n ayant la signification donnée ci-après,

* $R_2$ et $R_3$ représentent un atome d'hydrogène, un radical alkyle linéaire ou ramifié ayant de 1 à 20 atomes de carbone, un radical $-OR_6$ ou un radical $-SR_6$, $R_6$ ayant la signification donnée ci-après, étant entendu que $R_2$ et $R_3$, pris ensemble, peuvent former avec le cycle aromatique adjacent un cycle à 5 ou 6 chainons éventuellement substitué par des groupes méthyle et/ou éventuellement interrompu par un atome d'oxygène ou de soufre,

* $R_4$ représente un atome d'hydrogène, un atome d'halogène, un radical alkyle inférieur ou un radical $-OR_6$, $R_6$ ayant la signification donnée ci-après,

étant entendu que dans tout ce qui précède :

- $R_5$ a la même signification que $R_4$,
- $R_6$ représente un atome d'hydrogène, un radical alkyle linéaire ou ramifié ayant de 1 à 20 atomes de carbone ou un radical $-CO-R_9$, $R_9$ ayant la signification donnée ci-après,
- $R_7$ représente :

  (a) un atome d'hydrogène
  (b) un radical alkyle inférieur
  (c) un radical de formule :

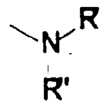

  R' et R'' ayant la signification donnée ci-après,
  (d) un radical $-OR_8$, $R_8$ ayant la signification donnée ci-après,

- $R_8$ représente un atome d'hydrogène, un radical alkyle linéaire ou ramifié ayant de 1 à 20 atomes de carbone, un radical alkényle, un radical mono ou polyhydroxyalkyle, un radical aryle ou aralkyle éventuellement substitué ou un reste de sucre ou un reste d'aminoacide ou de peptide,
- $R_9$ représente un radical alkyle inférieur,
- $R_{10}$ représente un atome d'hydrogène ou un radical alkyle inférieur,
- $R_{11}$ représente un atome d'hydrogène, un radical alkyle inférieur, un radical $-CO-R_9$ ou un radical $-COOR_9$,
- $R_{12}$ représente un radical $R_6$ ou un radical $-CH_2-O-CH_2-CH_2-O-CH_3$,
- $R_{13}$ représente un atome d'hydrogène ou un radical alkyle inférieur,
- $R_{14}$ représente un radical $-OR_8$ ou un radical de formule

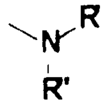

  R' et R'' ayant la signification ci-dessous,

- R' et R'' représentent un atome d'hydrogène, un radical alkyle inférieur, un radical mono ou polyhydroxyalkyle, un radical aryle éventuellement substitué ou un reste d'aminoacide ou de peptide ou de sucre ou encore, pris ensemble, forment un hétérocycle,
- t est un nombre entier égal à 0,1 ou 2,
- n est un nombre entier égal à 0 ou 1,

- les radicaux X et Y ci-dessus, pris ensemble, peuvent former un radical unique à double liaison de formule $=N-OR_6$ ou $=CH-COR_{14}$, ainsi que leurs sels, et leurs isomères optiques et géométriques.

**2.** Composés selon la revendication 1, caractérisés en ce qu'ils se présentent sous forme de sels d'un métal alcalin ou alcalino-terreux, ou encore de zinc ou d'une amine organique.

**3.** Composés selon l'une des revendications 1 ou 2, caractérisés en ce que les radicaux alkyles inférieurs sont choisis dans le groupe constitué par les radicaux méthyle, éthyle, isopropyle, butyle, tertiobutyle et hexyle.

**4.** Composés selon l'une quelconque des revendications précédentes, caractérisés en ce que les radicaux alkyles linéaires ou ramifiés ayant de 1 à 15 atomes de carbone sont choisis dans le groupe constitué par les radicaux méthyle, éthyle, propyle, 2-éthyl-hexyle, octyle, dodécyle, hexadécyle et octadécyl.

**5.** Composés selon l'une quelconque des revendications précédentes, caractérisés en ce que les radicaux monohydroxyalkyles sont choisis dans le groupe constitué par les radicaux 2-hydroxyéthyle, 2-hydroxypropyle ou 3-hydroxypropyle.

**6.** Composés selon l'une quelconque des revendications précédentes, caractérisés en ce que les radicaux polyhydroxyalkyles sont choisis dans le groupe constitué par les radicaux 2,3-dihydroxypropyle, 2,3,4-trihydroxybutyle, 2,3,4,5-tétrahydroxypentyle ou le reste du pentaérythritol.

**7.** Composés selon l'une quelconque des revendications précédentes, caractérisés en ce que le radical aryle est un radical phényle éventuellement substitué par au moins un atome d'halogène, un hydroxyle ou une fonction nitro.

**8.** Composés selon l'une quelconque des revendications précédentes, caractérisés en ce que les radicaux aralkyles sont choisis dans le groupe constitué par les radicauxl benzyle ou phénéthyle éventuellement substitués par au moins un atome d'halogène, un hydroxyle ou une fonction nitro.

**9.** Composés selon l'une quelconque des revendications précédentes, caractérisés en ce que les radicaux alkényles sont choisis dans le groupe constitué par les radicaux contenant de 1 à 5 atomes de carbone et présentant une ou plusieurs insaturations éthyléniques, en particulier le radical allyle.

**10.** Composés selon l'une quelconque des revendications précédentes, caractérisés en ce que les restes de sucre sont choisis dans le groupe constitué par les restes de glucose, de galactose, de mannose ou d'acide glucuronique.

**11.** Composés selon l'une quelconque des revendications précédentes, caractérisés en ce que les restes d'aminoacide sont choisis dans le groupe constitué par les restes dérivant de la lysine, de la glycine ou de l'acide aspartique.

**12.** Composés selon l'une quelconque des revendications précédentes, caractérisés en ce que les restes de peptides sont choisis dans le groupe constitué par les restes de dipeptides ou de tripeptides.

**13.** Composés selon l'une quelconque des revendications précédentes, caractérisés en ce que les radicaux hétérocycliques sont choisis dans le groupe constitué par les radicaux pipéridino, morpholino, pyrrolidino ou pipérazino, éventuellement substitués en position 4 par un radical alkyle en $C_1$-$C_6$ ou mono ou polyhydroxyalkyle.

**14.** Composés selon l'une quelconque des revendications précédentes, caractérisés en ce que les atomes d'halogènes sont choisis dans le groupe constitué par le fluor, le chlore et le brome.

**15.** Composés selon la revendication 1, caractérisés par le fait qu'ils sont pris dans le groupe constitué par :

- Acide 4-[α-amino-(5,6,7,8-tétrahydro-5,5,8,8-tétraméthyl-2-naphtyl)acétamido]benzoïque
- Acide 4-[α-méthoximino-(5,6,7,8-tétrahydro-5,5,8,8-tétraméthyl-2-naphtyl)acétamido]benzoïque
- Acide 4-[α-acétamido-(5,6,7,8-tétrahydro-5,5,8,8-tétraméthyl-2-naphtyl)acétamido]benzoïque
- Acide *anti*-4-[α-hydroximino-(5,6,7,8-tétrahydro-5,5,8,8-tétraméthyl-2-naphtyl)acétamido]benzoïque

- Acide *syn*-4-[α-hydroximino-(5,6,7,8-tétrahydro-5,5,8,8-tétraméthyl-2-naphtyl)acétamido]benzoïque
- Acide 4-[α-méthoxycarbonyl-(5,6,7,8-tétrahydro-5,5,8,8-tétraméthyl-2-naphtyl)acétamido]benzoïque
- Acide 4-[α-carboxy-(5,6,7,8-tétrahydro-5,5,8,8-tétraméthyl-2-naphtyl)acétamido]benzoïque
- Acide 4-[α-carbamoyl-(5,6,7,8-tétrahydro-5,5,8,8-tétraméthyl-2-naphtyl)acétamido]benzoîque
- Acide 2-hydroxy-4-[α-*tert*-butoxycarboxamido-(5,6,7,8-tétrahydro-5,5,8,8-tétraméthyl-2-naphtyl)acétamido] benzoïque
- Acide 2-hydroxy-4-[α-amino-(5,6,7,8-tétrahydro-5,5,8,8-tétraméthyl-2-naphtyl)acétamido]benzoïque
- Acide 4-[α-méthoxycarbonyl-(5,6,7,8-tétrahydro-5,5,8,8-tétraméthyl-2-naphtyl)méthylcarbamoyl]benzoïque
- Acide 4-[α-carboxyl-(5,6,7,8-tétrahydro-5,5,8,8-tétraméthyl-2-naphtyl)méthylcarbamoyl]benzoïque
- Acide 4-[α-hydroxyméthyl-(5,6,7,8-tétrahydro-5,5,8,8-tétraméthyl-2-naphtyl)méthylcarbamoyl]benzoïque
- Acide -4-[α-acétoxyméthyl-(5,6,7,8-tétrahydro-5,5,8,8-tétraméthyl-2-naphtyl)méthylcarbamoyl]benzoïque
- Acide syn-4-[α-propyloxyimino-(5,6,7,8-tétrahydro-5,5,8,8-tétraméthyl-2-naphtyl)acétamido]benzoïque
- Acide anti 4-[α-propyloxyimino-(5,6,7,8-tétrahydro-5,5,8,8-tétraméthyl-2-naphtyl)acétamido]benzoïque
- Acide syn-4-(α-hexyloxyimino-(5,6,7,8-tétrahydro-5,5,8,8-tétraméthyl-2-naphtyl)acétamido]benzoïque
- Acide anti 4-[α-hexyloxyimino-(5,6,7,8-tétrahydro-5,5,8,8-tétraméthyl-2-naphtyl)acétamido]benzoïque
- Acide syn-4-[α-heptyloxyimino-(5,6,7,8-tétrahydro-5,5,8,8-tétraméthyl-2-naphtyl)acétamido]benzoïque
- Acide anti 4-[α-heptyloxyimino-(5,6,7,8-tétrahydro-5,5,8,8-tétraméthyl-2-naphtyl)acétamido]benzoïque
- Acide syn-4-[α-nonyloxyimino-(5,6,7,8-tétrahydro-5,5,8,8-tétraméthyl-2-naphtyl)acétamido]benzoïque
- Acide anti 4-[α-nonyloxyimino-(5,6,7,8-tétrahydro-5,5,8,8-tétraméthyl-2-naphtyl)acétamido]benzoïque
- Acide syn-4-[a-dodecyloxyimino-(5,6,7,8-tétrahydro-5,5,8,8-tétraméthyl-2-naphtyl)acétamido]benzoïque
- 5-[α-amino-(5,6,7,8-tétrahydro-5,5,8,8-tétraméthyl-2-naphtyl)acétamido]-2-thiophènecarboxylate de méthyle
- N-méthyl-5-[α-amino-(5,6,7,8-tétrahydro-5,5,8,8-tétraméthyl-2-naphtyl) acétamido]-2-pyrrolecarboxylate de méthyle
- N-méthyl-4-[α-amino-(5,6,7,8-tétrahydro-5,5,8,8-tétraméthyl-2-naphtyl) acétamido]-2-pyrrolecarboxylate de méthyle
- N-propyl-4-[α-amino-(5,6,7,8-tétrahydro-5,5,8,8-tétraméthyl-2-naphtyl) acétamido]-2-pyrrolecarboxylate de méthyle
- 4'-[α-amino-(5,6,7,8-tétrahydro-5,5,8,8-tétraméthyl-2-naphtyl)acétamido] acétophénone
- 4-[α-amino-(5,6,7,8-tétrahydro-5,5,8,8-tétraméthyl-2-naphtyl)toluène
- 4-[α-amino-(5,6,7,8-tétrahydro-5,5,8,8-tétraméthyl-2-naphtyl)phényl sulfonylméthane
- 4-[α-tert-butoxycarboxamido-(5,6,7,8-tétrahydro-5,5,8,8-tétraméthyl-2-naphtyl)acétamido]phénylcarboxamide

- Acétate de 4'-[α-amino-(5,6,7,8-tétrahydro-5,5,8,8-tétraméthyl-2-naphtyl)benzyle
- Acide syn-2-hydroxy-4-[α-hydroxyimino-(5,6,7,8-tétrahydro-5,5,8,8-tétraméthyl-2-naphtyl)acétamido]benzoïque
- Acide 4-[2-éthoxycarbonyl-1-(5,6,7,8-tétrahydro-5,5,8,8-tétraméthyl-2-naphtyl) acrylamido]benzoïque
- 4-[2-tert-butoxycarbonyl-1-(5,6,7,8-tétrahydro-5,5,8,8-tétraméthyl-2-naphtyl) acrylamido]benzoate d'allyle

**16.** Composés selon la revendication 1, caractérisés en ce que X représente l'atome d'hydrogène, Y représente le radical $-NH_2$, et $R_7$ représente un radical $-OR_8$, ces deux derniers radicaux ayant la signification donnée ci-avant.

**17.** Composés selon l'une quelconque des revendications précédentes pour une utilisation comme médicament.

**18.** Composés selon la revendication 17 pour une utilisation comme médicament destiné au traitement des affections dermatologiques, rhumatismales, respiratoires, cardiovasculires et ophtalmologiques.

**19.** Utilisation de l'un au moins des composés définis aux revendications 1 à 16 pour la fabrication d'un médicament destiné au traitement des affections dermatologiques, rhumatismales, respiratoires, cardiovasculires et ophtalmologiques.

**20.** Composition pharmaceutique, caractérisée par le fait qu'elle comprend, dans un support pharmaceutiquement acceptable, au moins un des composés tels que définis à l'une quelconque des revendications 1 à 16.

**21.** Composition selon la revendication 20, caractérisée en ce que la concentration en composé(s) selon l'une des revendication 1 à 16 est comprise entre 0,001 % et 5 % en poids par rapport à l'ensemble de la composition.

**22.** Composition cosmétique, caractérisée par le fait qu'elle comprend, dans un support cosmétiquement acceptable, au moins un des composés tels que définis à l'une quelconque des revendications 1 à 16.

**23.** Composition selon la revendication 22, caractérisée en ce que la concentration en composé(s) selon l'une des revendications 1 à 16 est comprise entre 0,001 % et 3 % en poids par rapport à l'ensemble de la composition.

**24.** Utilisation d'une composition cosmétique telle que définie à l'une des revendications 22 ou 23 pour l'hygiène corporelle ou capillaire.

**Patentansprüche**

**1.** Biaromatische von Amiden abgeleitete Verbindungen, dadurch gekennzeichnet, daß sie der folgenden allgemeinen Formel I entsprechen:

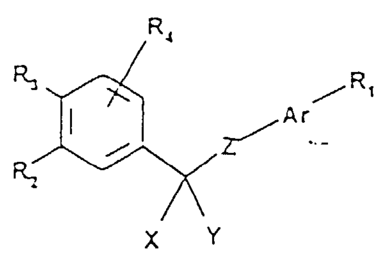

(I),

worin bedeuten:

. Z -CO-NH- oder -NH-CO-,
. Ar eine Gruppe, die unter den Gruppen der folgenden Formeln (a)-(e) ausgewählt ist:

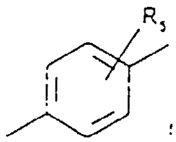 (a) 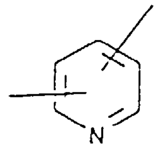 (b)

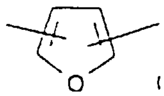 (c) 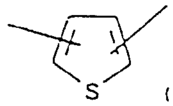 (d) 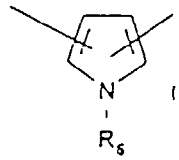 (e) ,

wobei $R_5$ und $R_6$ die nachstehend angegebenen Bedeutungen aufweisen,

. $R_1$

(i) ein Wasserstoffatom
(ii) eine Gruppe $-CH_3$
(iii) eine Gruppe $-CH_2-O-R_6$
(iv) eine Gruppe $-O-R_6$
(v) eine Gruppe $-CO-R_7$
(vi) eine Gruppe $-S(O)_tR_9$,
wobei $R_6$, $R_7$, $R_9$ und t die nachstehend angegebenen Bedeutungen aufweisen,

. X Wasserstoff oder eine niedere Alkylgruppe,

. Y

(i) eine Gruppe der Formel

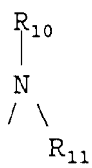

(ii) eine Gruppe $-CH_2OR_{12}$
(iii) eine Gruppe $-COR_{13}$
(iv) eine Gruppe $-(CH_2)_nCOR_{14}$,
wobei $R_{10}$, $R_{11}$, $R_{12}$, $R_{13}$, $R_{14}$ und n die nachstehend angegebenen Bedeutungen aufweisen,

. $R_2$ und $R_3$ Wasserstoff, eine geradkettige oder verzweigte Alkylgruppe mit 1 bis 20 Kohlenstoffatomen, eine Gruppe $-OR_6$ oder eine Gruppe $-SR_6$, worin die nachfolgend angegebene Bedeutung aufweist, wobei $R_2$ und $R_3$ gemeinsam mit dem angrenzenden aromatischen Ring einen 5- oder 6-gliedrigen Ring bilden können, der ggf. mit Methylgruppen substituiert sein kann und/oder ggf. ein Sauerstoff- oder Schwefelatom als Ringatom enthält,

. $R_4$ Wasserstoff, Halogen, eine niedere Alkylgruppe oder eine Gruppe $-OR_6$, wobei $R_6$ die nachfolgend angegebene Bedeutung aufweist,
mit der Maßgabe, daß im folgenden:

- $R_5$ die gleiche Bedeutung wie $R_4$ aufweist,
- $R_6$ Wasserstoff, eine geradkettige oder verzweigte Alkylgruppe mit 1 bis 20 Kohlenstoffatomen oder eine Gruppe $-CO-R_9$ bedeutet, wobei $R_9$ die nachfolgend angegebene Bedeutung aufweist,
- $R_7$ bedeutet:

(a) Wasserstoff
(b) eine niedere Alkylgruppe
(c) eine Gruppe der Formel

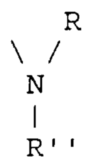

,

wobei R' und R'' die nachfolgend angegebenen Bedeutungen aufweisen,
(d) eine Gruppe $-OR_8$, wobei $R_8$ die nachfolgend angegebene Bedeutung aufweist,

- $R_8$ Wasserstoff, eine geradkettige oder verzweigte Alkylgruppe mit 1 bis 20 Kohlenstoffatomen, eine Alkenylgruppe, eine Mono- oder Polyhydroxyalkylgruppe, eine Arylgruppe oder Aralkylgruppe, die ggf. substituiert sind, oder einen Zucker- oder Aminosäure- oder Peptidrest bedeutet,
- $R_9$ eine niedere Alkylgruppe bedeutet,
- $R_{10}$ Wasserstoff oder eine niedere Alkylgruppe bedeutet,
- $R_{11}$ Wasserstoff, eine niedere Alkylgruppe, eine Gruppe $-CO-R_9$ oder eine Gruppe $-COOR_9$ bedeutet,
- $R_{12}$ eine Gruppe $R_6$ oder eine Gruppe $-CH_2-O-CH_2-CH_2-O-$ bedeutet,
- $R_{13}$ Wasserstoff oder eine niedere Alkylgruppe bedeutet,
- $R_{14}$ $-OR_8$ oder eine Gruppe der Formel

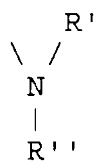

,

bedeutet, wobei R' und R'' die nachfolgenden Bedeutungen aufweisen,
- R' und R'' Wasserstoff, eine niedere Alkylgruppe, eine Mono- oder Polyhydroxyalkylgruppe, eine ggf. substituierte Arylgruppe oder einen Aminosäure- oder Peptid- oder Zuckerrest bedeuten oder R' und R'' gemeinsam einen Heterocyclus bilden,
- t Null oder eine ganze Zahl 1 oder 2 bedeutet,
- n Null oder 1 bedeutet
und
- die oben genannten Gruppen X und Y gemeinsam eine einzige Gruppe mit Doppelbindung der Formel $=N-OR_6$ oder $=CH-COR_{14}$ bilden können,

sowie deren Salze und optischen und geometrischen Isomeren.

**2.** Verbindungen nach Anspruch 1, dadurch gekennzeichnet, daß sie in Form von Alkali- oder Erdalkalimetallsalzen oder auch als Zinksalze oder organische Aminsalze vorliegen.

**3.** Verbindungen nach einem der Ansprüche 1 oder 2, dadurch gekennzeichnet, daß die niederen Alkylgruppen unter den Gruppen Methyl, Ethyl, Isopropyl, Butyl, *tert.*-Butyl und Hexyl ausgewählt sind.

**4.** Verbindungen nach einem der vorhergehenden Ansprüche, dadurch gekennzeichnet, daß die geradkettigen oder verzweigten Alkylgruppen mit 1 bis 15 Kohlenstoffatomen unter den Gruppen Methyl, Ethyl, Propyl, 2-Ethylhexyl, Octyl, Dodecyl, Hexadecyl und Octadecyl ausgewählt sind.

**5.** Verbindungen nach einem der vorhergehenden Ansprüche, dadurch gekennzeichnet, daß die Monohydroxyalkylgruppen unter den Gruppen 2-Hydroxyethyl, 2-Hydroxypropyl oder 3-Hydroxypropyl ausgewählt sind.

6. Verbindungen nach einem der vorhergehenden Ansprüche, dadurch gekennzeichnet, daß die Polyhydroxyalkylgruppen unter den Gruppen 2,3-Dihydroxypropyl, 2,3,4-Trihydroxybutyl, 2,3,4,5-Tetrahydroxypentyl oder der Pentaerythritgruppe ausgewählt sind.

7. Verbindungen nach einem der vorhergehenden Ansprüche, dadurch gekennzeichnet, daß die Arylgruppe eine Phenylgruppe ist, die ggf. mit mindestens einem Halogenatom, einer Hydroxygruppe oder einer Nitrogruppe substituiert ist.

8. Verbindungen nach einem der vorhergehenden Ansprüche, dadurch gekennzeichnet, daß die Aralkylgruppen unter den Gruppen Benzyl oder Phenethyl ausgewählt sind, die ggf. mit mindestens einem Halogenatom, einer Hydroxygruppe oder einer Nitrogruppe substituiert sind.

9. Verbindungen nach einem der vorhergehenden Ansprüche, dadurch gekennzeichnet, daß die Alkenylgruppen unter den Gruppen ausgewählt sind, die 1 bis 5 Kohlenstoffatome enthalten und eine oder mehrere ethylenisch ungesättigte Bindungen enthalten, insbesondere Allyl.

10. Verbindungen nach einem der vorhergehenden Ansprüche, dadurch gekennzeichnet, daß die Zuckergruppen unter den Resten von Glucose, Galactose, Mannose und Glucuronsäure ausgewählt sind.

11. Verbindungen nach einem der vorhergehenden Ansprüche, dadurch gekennzeichnet, daß die Aminosäuregruppen unter den Gruppen ausgewählt sind, die von Lysin, Glycin oder Asparaginsäure stammen.

12. Verbindungen nach einem der vorhergehenden Ansprüche, dadurch gekennzeichnet, daß die Peptidgruppen unter den Dipeptid- oder Tripeptidresten ausgewählt sind.

13. Verbindungen nach einem der vorhergehenden Ansprüche, dadurch gekennzeichnet, daß die heterocyclichen Gruppen unter den Gruppen Piperidino, Morpholino, Pyrrolidino oder Piperazino ausgewählt sind, die ggf. in 4-Stellung mit einer Alkylgruppe mit 1 bis 6 Kohlenstoffatomen oder einer Mono- oder Polyhydroxyalkylgruppe substituiert sind.

14. Verbindungen nach einem der vorhergehenden Ansprüche, dadurch gekennzeichnet, daß die Halogenatome unter Fluor, Chlor und Brom ausgewählt sind.

15. Verbindungen nach Anspruch 1, dadurch gekennzeichnet, daß sie ausgewählt sind unter:

- 4-[α-Amino-(5,6,7,8-tetrahydro-5,5,8,8-tetramethyl-2-naphthyl)-acetamido]-benzoesäure
- 4-[α-Methoximino-(5,6,7,8-tetrahydro-5,5,8,8-tetramethyl-2-naphthyl)-acetamido]-benzoesäure
- 4-[α-Acetamido-(5,6,7,8-tetrahydro-5,5,8,8-tetramethyl-2-naphthyl)-acetamido]-benzoesäure
- *anti*-4-[α-Hydroxyimino-(5,6,7,8-tetrahydro-5,5,8,8-tetramethyl-2-naphthyl)-acetamido]-benzoesäure
- *syn*-4-[α-Hydroxyimino-(5,6,7,8-tetrahydro-5,5,8,8-tetramethyl-2-naphthyl)-acetamido]-benzoesäure
- 4-[α-Methoxycarbonyl-(5,6,7,8-tetrahydro-5,5,8,8-tetramethyl-2-naphthyl)-acetamido]-benzoesäure
- 4-[α-Carboxy-(5,6,7,8-tetrahydro-5,5,8,8-tetramethyl-2-naphthyl)-acetamido]-benzoesäure
- 4-[α-Carbamoyl-(5,6,7,8-tetrahydro-5,5,8,8-tetramethyl-2-naphthyl)-acetamido]-benzoesäure
- 2-Hydroxy-4-[α-*tert.*-butoxycarboxamido- (5,6,7,8-tetrahydro-5,5,8,8-tetramethyl-2-naphthyl)-acet-amido]-benzoesäure
- 2-Hydroxy-4-[α-amino-(5,6,7,8-tetrahydro-5,5,8,8-tetramethyl-2-naphthyl)-acetamido]-benzoesäure
- 4-[α-Methoxycarbonyl-(5,6,7,8-tetrahydro-5,5,8,8-tetramethyl-2-naphthyl)-methylcarbamoyl]-benzoesäure
- 4-[α-Carboxy-(5,6,7,8-tetrahydro-5,5,8,8-tetra-methyl-2-naphthyl)-methylcarbamoyl]-benzoesäure
- 4-[α-Hydroxymethyl-(5,6,7,8-tetrahydro-5,5,8,8-tetramethyl-2-naphthyl)-methylcarbamoyl]-benzoesäure
- 4-[α-Acetoxymethyl-(5,6,7,8-tetrahydro-5,5,8,8-tetramethyl-2-naphthyl)-methylcarbamoyl]-benzoesäure
- *syn*-4-[α-Propyloxyimino-(5,6,7,8-tetrahydro-5,5,8,8-tetramethyl-2-naphthyl)-acetamidol-benzoesäure
- *anti*-4-[α-Propyloxyimino-(5,6,7,8-tetrahydro-5,5,8,8-tetramethyl-2-naphthyl)-acetamido]-benzoesäure
- *syn*-4-[α-Hexyloxyimino-(5,6,7,8-tetrahydro-5,5,8,8-tetramethyl-2-naphthyl)-acetamido]-benzoesäure
- *anti*-4-[α-Hexyloxyimino-(5,6,7,8-tetrahydro-5,5,8,8-tetramethyl-2-naphthyl)-acetamido]-benzoesäure
- *syn*-4-[α-Heptyloxyimino-(5,6,7,8-tetrahydro-5,5,8,8-tetramethyl-2-naphthyl)-acetamido]-benzoesäure
- *anti*-4-[α-Heptyloxyimino-(5,6,7,8-tetrahydro-5,5,8,8-tetramethyl-2-naphthyl)-acetamido]-benzoesäure
- *syn*-4-[α-Nonyloxyimino-(5,6,7,8-tetrahydro-5,5,8,8-tetramethyl-2-naphthyl)-acetamido]-benzoesäure
- *anti*-4-[α-Nonyloxyimino-(5,6,7,8-tetrahydro-5,5,8,8-tetramethyl-2-naphthyl)-acetamido]-benzoesäure

- *syn*-4-[α-Dodecyloxyimino-(5,6,7,8-tetrahydro-5,5,8,8-tetramethyl-2-naphthyl)-acetamido]-benzoesäure
- 5-[α-Amino-(5,6,7,8-tetrahydro-5,5,8,8-tetramethyl-2-naphthyl)-acetamido]-2-thiophencarbonsäuremethylester
- N-Methyl-5-[α-amino-(5,6,7,8-tetrahydro-5,5,8,8-tetramethyl-2-naphthyl)-acetamido]-2-pyrrolcarbonsäuremethylester
- N-Methyl-4-[α-amino-(5,6,7,8-tetrahydro-5,5,8,8-tetramethyl-2-naphthyl)-acetamido]-2-pyrrolcarbonsäuremethylester
- N-Propyl-4-[α-amino-(5,6,7,8-tetrahydro-5,5,8,8-tetramethyl-2-naphthyl)-acetamido]-2-pyrrolcarbonsäuremethylester
- 4'-[α-Amino-(5,6,7,8-tetrahydro-5,5,8,8-tetramethyl2-naphthyl)-acetamido]-acetophenon
- 4-[α-Amino-(5,6,7,8-tetrahydro-5,5,8,8-tetramethyl-2-naphthyl)-toluol
- 4-[α-Amino-(5,6,7,8-tetrahydro-5,5,8,8-tetramethyl-2-naphthyl)-phenylsulfonylmethan
- 4-[α-*tert.*-Butoxycarboxamid-(5,6,7,8-tetrahydro-5,5,8,8-tetramethyl-2-naphthyl)-acetamido]-phenylcarboxamid
- 4'-[α-Amino-(5,6,7,8-tetrahydro-5,5,8,8-tetramethyl-2-naphthyl)]-benzylacetat
- *syn*-2-Hydroxy-4-[α-hydroxyimino-(5,6,7,8-tetrahydro-5,5,8,8-tetramethyl-2-naphthyl)-acetamido]-benzoesäure
- 4-[2-Ethoxycarbonyl-1-(5,6,7,8-tetrahydro-5,5,8,8-tetramethyl-2-naphthyl)-acrylamido]-benzoesäure
- 4-(2-*tert.*-Butoxycarbonyl-1-(5,6,7,8-tetrahydro-5,5,8,8-tetramethyl-2-naphthyl)-acrylamido]-benzoesäureallylester.

**16.** Verbindungen nach Anspruch 1, dadurch gekennzeichnet, daß X Wasserstoff, Y die Gruppe $-NH_2$ und $R_7$ die Gruppe - $OR_8$ bedeutet, wobei die beiden letzten Gruppen die oben angegebenen Bedeutungen aufweisen.

**17.** Verbindungen nach einem der vorhergehenden Ansprüche für eine Verwendung als Arzneimittel.

**18.** Verbindungen nach Anspruch 17 für eine Verwendung als Arzneimittel, das zur Behandlung von dermatologischen Erkrankungen, rheumatischen Erkrankungen, Erkrankungen der Atemwege, kardiovaskulären Erkrankungen und opthalmologischen Erkrankungen bestimmt ist.

**19.** Verwendung mindestens einer Verbindung nach Anspruch 1 bis 16 zur Herstellung eines Arzneimittels, das zur Behandlung von dermatologischen Erkrankungen, rheumatischen Erkrankungen, Erkrankungen der Atemwege, kardiovaskulären Erkrankungen und ophthalmologischen Erkrankungen bestimmt ist.

**20.** Pharmazeutische Zusammensetzung, dadurch gekennzeichnet, daß sie in einem pharmazeutisch akzeptablen Träger mindestens eine der Verbindungen nach einem der Ansprüche 1 bis 16 enthält.

**21.** Zusammensetzung nach Anspruch 20, dadurch gekennzeichnet, daß die Konzentration der Verbindung(en) nach einem der Ansprüche 1 bis 16 im Bereich von 0,001 bis 5 Gew.-%, bezogen auf die gesamte Zusammensetzung, liegt.

**22.** Kosmetische Zusammensetzung, dadurch gekennzeichnet, daß sie in einem kosmetisch akzeptablen Träger mindestens eine der Verbindungen nach einem der Ansprüche 1 bis 16 enthält.

**23.** Zusammensetzung nach Anspruch 22, dadurch gekennzeichnet, daß die Konzentration der Verbindung(en) nach einem der Ansprüche 1 bis 16 im Bereich von 0,001 bis 3 Gew.-%, bezogen auf die gesamte Zusammensetzung, liegt.

**24.** Verwendung einer kosmetischen Zusammensetzung nach einem der Ansprüche 22 oder 23 zur Körperpflege oder Haarpflege.

## Claims

**1.** Amide-derived biaromatic compounds, characterized by the fact that they correspond to the following general formula (I):

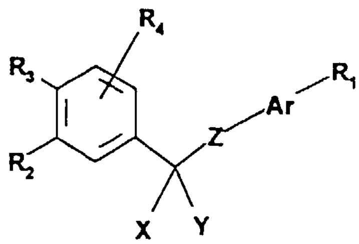

(I)

in which:

* Z represents -CO-NH- or -NH-CO-,
* Ar represents a radical chosen from the radicals of the following formulae (a)-(e)

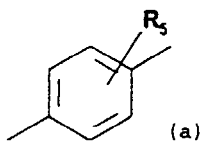

(a)

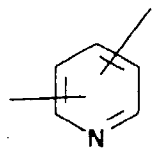

(b)

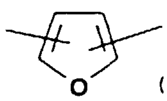

(c)

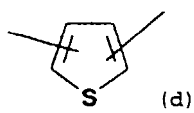

(d)

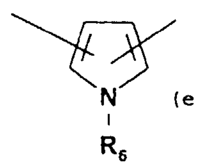

(e)

$R_5$ and $R_6$ having the meaning given below,

* $R_1$ represents: (i) a hydrogen atom

    (ii) a radical $-CH_3$
    (iii) a radical $-CH_2-O-R_6$
    (iv) a radical $-O-R_6$
    (v) a radical $-CO-R_7$,
    (vi) a radical $-S(O)_tR_9$

$R_6$, $R_7$, $R_9$ and t having the meaning given below,

* X represents a hydrogen atom or a lower alkyl radical,
* Y represents: (i) a radical of formula:

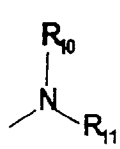

    (ii) a radical $-CH_2OR_{12}$
    (iii) a radical $-COR_{13}$
    (iv) a radical $-(CH_2)_n-COR_{14}$

$R_{10}$, $R_{11}$, $R_{12}$, $R_{13}$, $R_{14}$ and n having the meaning given below,

* $R_2$ and $R_3$ represent a hydrogen atom, a linear or branched alkyl radical having 1 to 20 carbon atoms, a radical $-OR_6$ or a radical $-SR_6$, $R_6$ having the meaning given below, it being understood that $R_2$ and $R_3$, taken together, can form with the adjacent aromatic ring a 5- or 6-membered ring optionally substituted by methyl groups and/or optionally interrupted by an oxygen or sulphur atom,
* $R_4$ represents a hydrogen atom, a halogen atom, a lower alkyl radical or a radical $-OR_6$, $R_6$ having the meaning given below,

it being understood that in all that precedes:

- $R_5$ has the same meaning as $R_4$,
- $R_6$ represents a hydrogen atom, a linear or branched alkyl radical having 1 to 20 carbon atoms or a radical $-CO-R_9$, $R_9$ having the meaning given below,
- $R_7$ represents:

  (a) a hydrogen atom
  (b) a lower alkyl radical
  (c) a radical of formula:

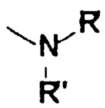

  R' and R" having the meaning given below,
  (d) a radical $-OR_8$, $R_8$ having the meaning given below,

- $R_8$ represents a hydrogen atom, a linear or branched alkyl radical having 1 to 20 carbon atoms, an alkenyl radical, a mono- or polyhydroxyalkyl radical, an optionally substituted aryl or aralkyl radical or a sugar residue or an amino acid or peptide residue,
- $R_9$ represents a lower alkyl radical,
- $R_{10}$ represents a hydrogen atom or a lower alkyl radical,
- $R_{11}$ represents a hydrogen atom, a lower alkyl radical, a radical $-CO-R_9$ or a radical $-COOR_9$,
- $R_{12}$ represents a radical $R_6$ or a radical $-CH_2-O-CH_2-CH_2-O-CH_3$,
- $R_{13}$ represents a hydrogen atom or a lower alkyl radical,
- $R_{14}$ represents a radical $-OR_8$ or a radical of formula

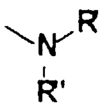

  R' and R" having the meaning below,
- R' and R" represent a hydrogen atom, a lower alkyl radical, a mono- or polyhydroxyalkyl radical, an optionally substituted aryl radical or an amino acid or peptide or sugar residue or alternatively, taken together, form a heterocycle,
- t is an integer equal to 0, 1 or 2,
- n is an integer equal to 0 or 1,
- the radicals X and Y above, taken together, can form a double bond-containing single radical of formula $=N-OR_6$ or $=CH-COR_{14}$, as well as salts thereof, and optical and geometric isomers thereof.

**2.** Compounds according to Claim 1, characterized in that they are in the form of salts of an alkali or alkaline-earth metal, or alternatively of zinc or of an organic amine.

**3.** Compounds according to one of Claims 1 or 2, characterized in that the lower alkyl radicals are chosen from the group consisting of methyl, ethyl, isopropyl, butyl, tert-butyl and hexyl radicals.

**4.** Compounds according to any one of the preceding claims, characterized in that the linear or branched alkyl radicals having 1 to 15 carbon atoms are chosen from the group consisting of methyl, ethyl, propyl, 2-ethylhexyl, octyl, dodecyl, hexadecyl and octadecyl radicals.

**5.** Compounds according to any one of the preceding claims, characterized in that the monohydroxyalkyl radicals are chosen from the group consisting of 2-hydroxyethyl, 2-hydroxypropyl or 3-hydroxypropyl radicals.

**6.** Compounds according to any one of the preceding claims, characterized in that the polyhydroxyalkyl radicals are chosen from the group consisting of 2,3-dihydroxypropyl, 2,3,4-trihydroxybutyl or 2,3,4,5-tetrahydroxypentyl radicals or the pentaerythritol residue.

**7.** Compounds according to any one of the preceding claims, characterized in that the aryl radical is a phenyl radical optionally substituted by at least one halogen atom, a hydroxyl or a nitro functional group.

**8.** Compounds according to any one of the preceding claims, characterized in that the aralkyl radicals are chosen from the group consisting of benzyl or phenethyl radicals optionally substituted by at least one halogen atom, a hydroxyl or a nitro functional group.

**9.** Compounds according to any one of the preceding claims, characterized in that the alkenyl radicals are chosen from the group consisting of the radicals containing 1 to 5 carbon atoms and having one or more ethylenic unsaturations, in particular the allyl radical.

**10.** Compounds according to any one of the preceding claims, characterized in that the sugar residues are chosen from the group consisting of glucose, galactose, mannose or glucuronic acid residues.

**11.** Compounds according to any one of the preceding claims, characterized in that the amino acid residues are chosen from the group consisting of residues derived from lysine, glycine or aspartic acid.

**12.** Compounds according to any one of the preceding claims, characterized in that the peptide residues are chosen from the group consisting of dipeptide or tripeptide residues.

**13.** Compounds according to any one of the preceding claims, characterized in that the heterocyclic radicals are chosen from the group consisting of piperidino, morpholino, pyrrolidino or piperazino radicals which are optionally substituted at position 4 by a $C_1$-$C_6$ alkyl radical or a mono- or polyhydroxyalkyl radical.

**14.** Compounds according to any one of the preceding claims, characterized in that the halogen atoms are chosen from the group consisting of fluorine, chlorine and bromine.

**15.** Compounds according to Claim 1, characterized by the fact that they are chosen from the group consisting of;

- 4 -[α-Amino-(5,6,7,8-tetrahydro-5,5,8,8-tetramethyl-2-naphthyl)acetamido]benzoic acid
- 4-[α-Methoxyimino-(5,6,7,8-tetrahydro-5,5,8,8-tetramethyl-2-naphthyl)acetamido]benzoic acid
- 4-[α-Acetamido-(5,6,7,8-tetrahydro-5,5,8,8-tetramethyl-2-naphthyl)acetamido]benzoic acid
- *anti*-4-[α-Hydroxyimino-(5,6,7,8-tetrahydro-5,5,8,8-tetramethyl-2-naphthyl)acetamido]benzoic acid
- *syn*-4-[α-Hydroxyimino-(5,6,7,8-tetrahydro-5,5,8,8-tetramethyl-2-naphthyl)acetamido]benzoic acid
- 4-[α-Methoxycarbonyl-(5,6,7,8-tetrahydro-5,5,8,8-tetramethyl-2-naphthyl)acetamido]benzoic acid
- 4-[α-Carboxy-(5,6,7,8-tetrahydro-5,5,8,8-tetramethyl-2-naphthyl)acetamido]benzoic acid
- 4-[α-Carbamoyl-(5,6,7,8-tetrahydro-5,5,8,8-tetramethyl-2-naphthyl)acetamido]benzoic acid
- 2-Hydroxy-4-[α-*tert*-butoxycarboxamido-(5,6,7,8-tetrahydro-5,5,8,8-tetramethyl-2-naphthyl)acetamido]benzoic acid
- 2-Hydroxy-4-[α-amino-(5,6,7,8-tetrahydro-5,5,8,8-tetramethyl-2-naphthyl)acetamido]benzoic acid
- 4-[α-Methoxycarbonyl-(5,6,7,8-tetrahydro-5,5,8,8-tetramethyl-2-naphthyl)methylcarbamoyl]benzoic acid
- 4-[α-Carboxyl-(5,6,7,8-tetrahydro-5,5,8,8-tetramethyl-2-naphthyl)methylcarbamoyl]benzoic acid
- 4-[α-Hydroxymethyl-(5,6,7,8-tetrahydro-5,5,8,8-tetramethyl-2-naphthyl)methylcarbamoyl]benzoic acid
- 4-[α-Acetoxymethyl-(5,6,7,8-tetrahydro-5,5,8,8-tetramethyl-2-naphthyl)methylcarbamoyl]benzoic acid
- *syn*-4-[α-Propyloxyimino-(5,6,7,8-tetrahydro-5,5,8,8-tetramethyl-2-naphthyl)acetamido]benzoic acid
- *anti*-4-[α-Propyloxyimino-(5,6,7,8-tetrahydro-5,5,8,8-tetramethyl-2-naphthyl)acetamido]benzoic acid
- *syn*-4-[α-hexyloxyimino-(5,6,7,8-tetrahydro-5,5,8,8-tetramethyl-2-naphthyl)acetamido]benzoic acid

- *anti*-4-[α-Hexyloxyimino-(5,6,7,8-tetrahydro-5,5,8,8-tetramethyl-2-naphthyl)acetamido]benzoic acid
- *syn*-4-[α-Heptyloxyimino-(5,6,7,8-tetrahydro-5,5,8,8-tetramethyl-2-naphthyl)acetamido]benzoic acid
- *anti*-4-[α-Heptyloxyimino-(5,6,7,8-tetrahydro-5,5,8,8-tetramethyl-2-naphthyl)acetamido]benzoic acid
- *syn*-4-[α-Nonyloxyimino-(5,6,7,8-tetrahydro-5,5,8,8-tetramethyl-2-naphthyl)acetamido]benzoic acid
- *anti*-4-[α-Nonyloxyimino-(5,6,7,8-tetrahydro-5,5,8,8-tetramethyl-2-naphthyl)acetamido]benzoic acid
- *syn*-4-[α-Dodecyloxyimino-(5,6,7,8-tetrahydro-5,5,8,8-tetramethyl-2-naphthyl)acetamido]benzoic acid
- Methyl 5-[α-amino-(5,6,7,8-tetrahydro-5,5,8,8-tetramethyl-2-naphthyl)acetamido]-2-thiophene-carboxylate
- Methyl N-methyl-5-[α-amino-(5,6,7,8-tetrahydro-5,5,8,8-tetramethyl-2-naphthyl)acetamido]-2-pyrrolecarboxylate
- Methyl N-methyl-4-[α-amino-(5,6,7,8-tetrahydro-5,5,8,8-tetramethyl-2-naphthyl)acetamido]-2-pyrrolecarboxylate
- Methyl N-propyl-4-[α-amino-(5,6,7,8-tetrahydro-5,5,8,8-tetramethyl-2-naphthyl)acetamido]-2-pyrrolecarboxylate
- 4'-[α-amino-(5,6,7,8-tetrahydro-5,5,8,8-tetramethyl-2-naphthyl)acetamido]acetophenone
- 4-[α-amino-(5,6,7,8-tetrahydro-5,5,8,8-tetramethyl-2-naphthyl)toluene
- 4-[α-amino-(5,6,7,8-tetrahydro-5,5,8,8-tetramethyl-2-naphthyl)phenyl sulphonylmethane
- 4 -[α-tert-butoxycarboxamido-(5,6,7,8-tetrahydro-5,5,8,8-tetramethyl-2-naphthyl)acetamidol-phenylcarboxamide
- 4'-[α-Amino-(5,6,7,8-tetrahydro-5,5,8,8-tetramethyl-2-naphthyl)benzyl acetate
- *syn*-2-Hydroxy-4-[α-hydroxyimino-(5,6,7,8-tetrahydro-5,5,8,8-tetramethyl-2-naphthyl)-acetamido]benzoic acid
- 4-[2-Ethoxycarbonyl-1-(5,6,7,8-tetrahydro-5,5,8,8-tetramethyl-2-naphthyl)acrylamido]benzoic acid
- Allyl 4-[2-tert-butoxycarbonyl-1-(5,6,7,8-tetrahydro-5,5,8,8-tetramethyl-2-naphthyl)acrylamido]benzoate.

**16.** Compounds according to Claim 1, characterized in that X represents a hydrogen atom, Y represents the $-NH_2$ radical, and $R_7$ represents a radical $-OR_8$, these last two radicals having the meaning given above.

**17.** Compounds according to any one of the preceding claims, for use as medicaments.

**18.** Compounds according to Claim 17, for use as medicaments for the treatment of dermatological, rheumatic, respiratory, cardiovascular and ophthalmological conditions.

**19.** Use of at least one of the compounds defined in Claims 1 to 16 for the manufacture of a medicament for the treatment of dermatological, rheumatic, respiratory, cardiovascular and ophthalmological conditions.

**20.** Pharmaceutical composition, characterized by the fact that it comprises, in a pharmaceutically acceptable carrier, at least one of the compounds as defined in any one of Claims 1 to 16.

**21.** Composition according to Claim 20, characterized in that the concentration of compound(s) according to one of Claims 1 to 16 is between 0.001 % and 5 % by weight relative to the whole composition.

**22.** Cosmetic composition, characterized by the fact that it comprises, in a cosmetically acceptable carrier, at least one of the compounds as defined in any one of Claims 1 to 16.

**23.** Composition according to Claim 22, characterized in that the concentration of compound(s) according to one of Claims 1 to 16 is between 0.001 % and 3 % by weight relative to the whole composition.

**24.** Use of a cosmetic composition as defined in one of Claims 22 or 23 for body or hair care.

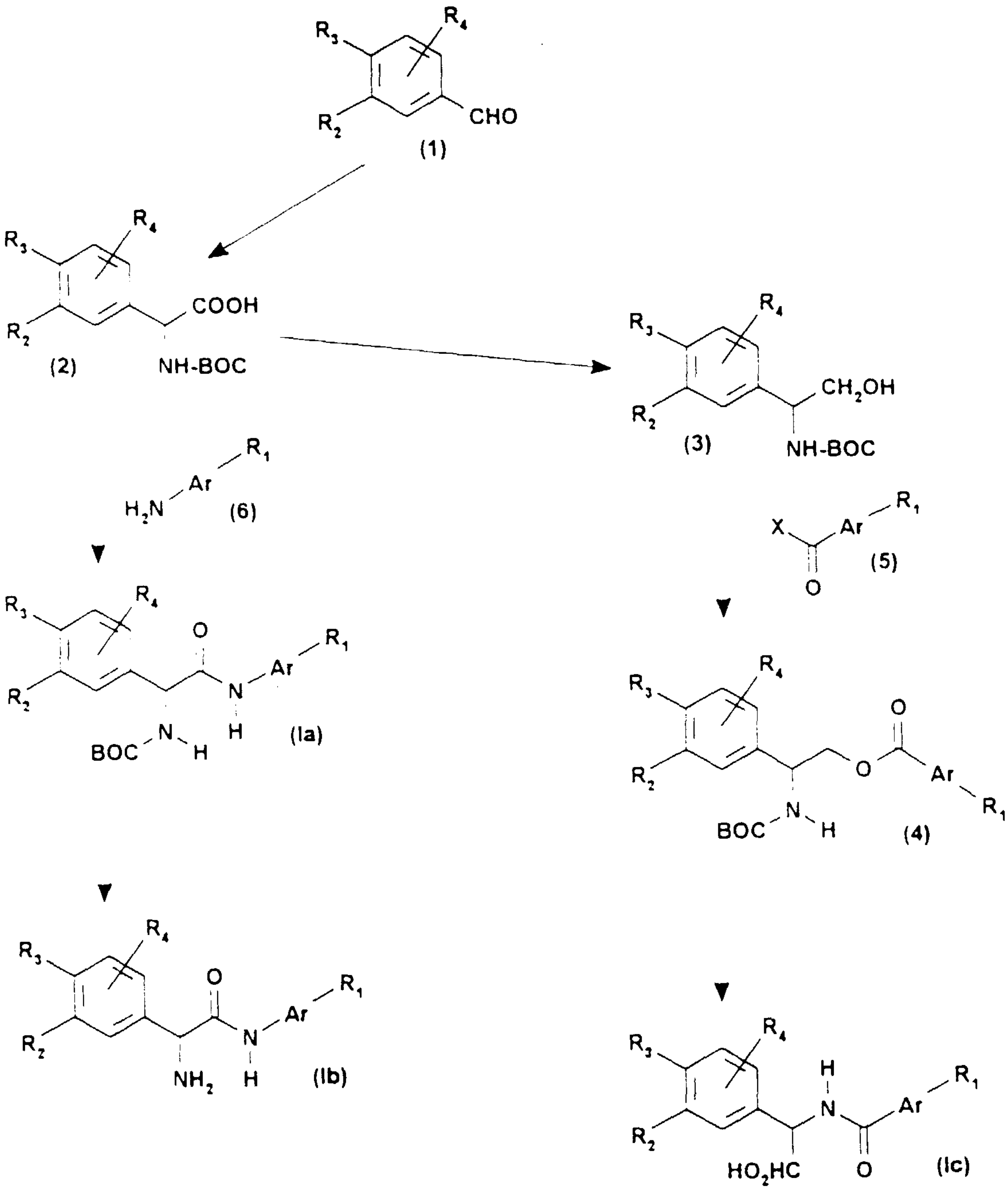

(1)

(2)

(3)

(6)

(5)

(Ia)

(4)

(Ib)

(Ic)

FIG.1

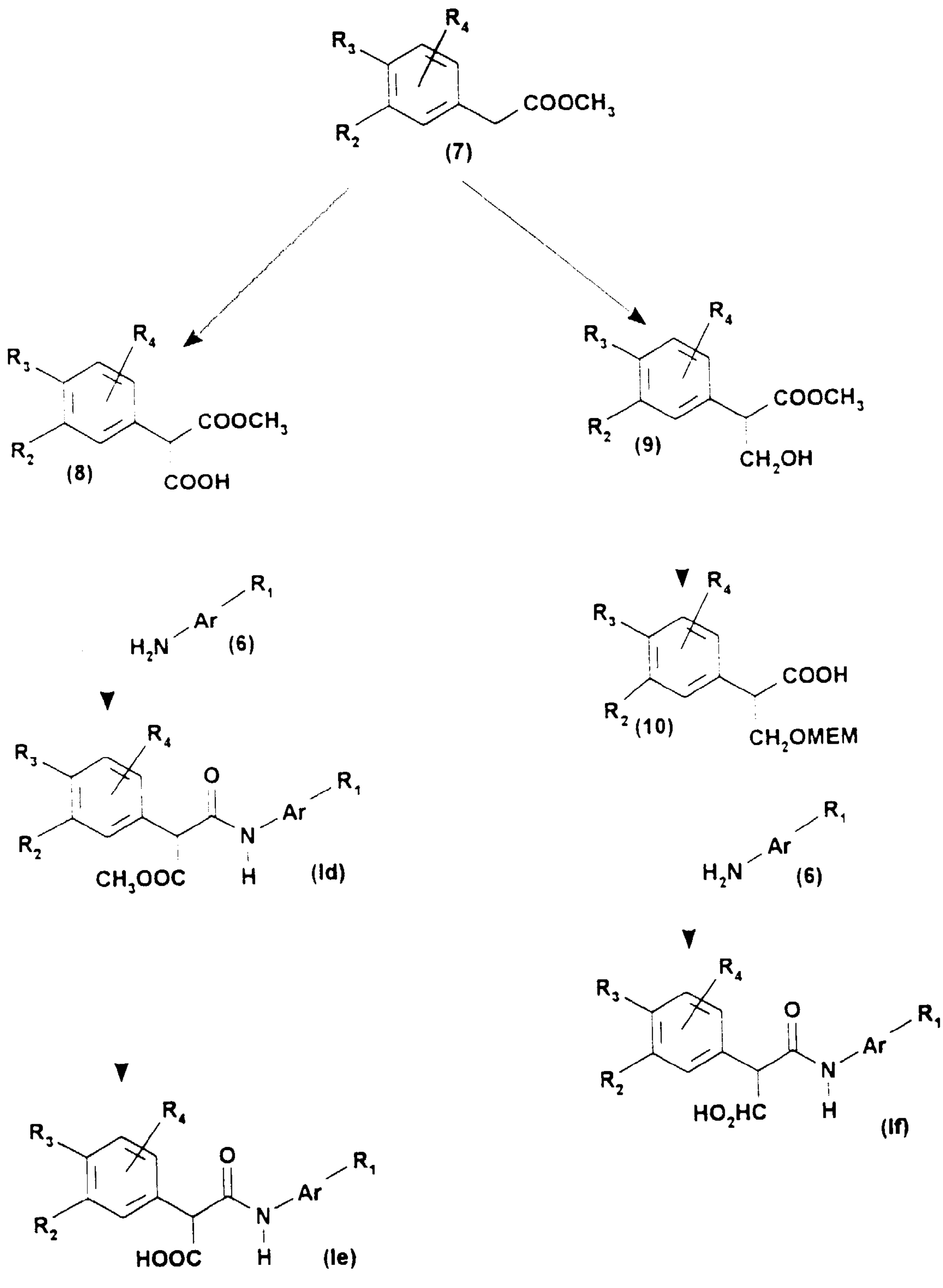

(7) (8) (9) (6) (10) (Id) (6) (If) (Ie)

FIG. 2